(19) Europäisches Patentamt
European Patent Office
Office européen des brevets

(11) **EP 1 879 909 B1**

(12) **EUROPÄISCHE PATENTSCHRIFT**

(45) Veröffentlichungstag und Bekanntmachung des Hinweises auf die Patenterteilung:
**29.10.2014 Patentblatt 2014/44**

(21) Anmeldenummer: **06763120.0**

(22) Anmeldetag: **11.05.2006**

(51) Int Cl.:
*A61P 7/00* *(2006.01)*   *A61K 47/12* *(2006.01)*
*A61K 47/18* *(2006.01)*

(86) Internationale Anmeldenummer:
**PCT/EP2006/062234**

(87) Internationale Veröffentlichungsnummer:
**WO 2006/120226 (16.11.2006 Gazette 2006/46)**

(54) **STABILISATORMOLEKÜLE-ABGEREICHERTE ALBUMINLÖSUNG**

STABILIZER MOLECULE-DEPLETED ALBUMIN SOLUTION

SOLUTION D'ALBUMINE APPAUVRIE EN MOLECULES STABILISATRICES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **13.05.2005 DE 102005023155**

(43) Veröffentlichungstag der Anmeldung:
**23.01.2008 Patentblatt 2008/04**

(73) Patentinhaber: **Albutec GmbH**
**18057 Rostock (DE)**

(72) Erfinder: **STANGE, Katrin**
**18059 Rostock (DE)**

(74) Vertreter: **WSL Patentanwälte Partnerschaft mbB**
**Postfach 6145**
**65051 Wiesbaden (DE)**

(56) Entgegenhaltungen:
**WO-A-96/37515       US-A1- 2004 217 055**
**US-A1- 2004 228 829**

- CHEN R F: "Removal of fatty acids from serum albumin by charcoal treatment." THE JOURNAL OF BIOLOGICAL CHEMISTRY. 25 JAN 1967, Bd. 242, Nr. 2, 25. Januar 1967 (1967-01-25), Seiten 173-181, XP002395537 ISSN: 0021-9258 in der Anmeldung erwähnt
- HUNTLEY T E ET AL: "BINDING PROPERTIES OF PURIFIED ADULT AND FETAL BOVINE SERUM ALBUMIN" BIOCHIMICA ET BIOPHYSICA ACTA, Bd. 490, Nr. 1, 1977, Seiten 112-119, XP002395538 ISSN: 0006-3002

**Beschreibung**

Die Erfindung betrifft ein Verfahren zur Herstellung einer wäßrigen Albuminlösung aus einer Albuminausgangslösung, welche Stabilisatormoleküle enthält, die in der Lage sind, Bindungsstellen des Albumins zu besetzen, wobei man bei dem Verfahren zur Erhöhung der Albuminbindungskapazität (ABiC) für andere Moleküle, z. B. solche mit physiologischen Effekten, wenigstens einen Teil der Stabilisatormoleküle von dem Albumin der Albuminausgangslösung entfernt und von der Albuminausgangslösung abtrennt.

HINTERGRUND DER ERFINDUNG

Bei zahlreichen schweren Erkrankungen mit daraus erwachsendem Organversagen spielt das Mißverhältnis zwischen der Volumenkapazität der Blutgefäße und dem in ihnen enthaltenen Füllvolumen eine zentrale Rolle. Ist die Volumenkapazität zu groß oder das Füllvolumen zu klein, resultieren Blutdruckabfall und zu geringe Durchblutung von Organen. Ist die Volumenkapazität zu klein oder das Füllvolumen zu groß, kommt es zu Blutdruckanstieg und in Folge dessen zu Herzinsuffizienz und/oder Lungenödem.

Für einen Blutdruckabfall mit Minderperfusionen können sowohl plötzliche als auch schleichende Volumenverluste in den Blutgefäßen verantwortlich sein. Plötzliche Volumenverluste können bei Blutungen auftreten. Schleichende Volumenverluste resultieren z. B. aus einem Flüssigkeitsverlust durch Transsudation aus dem Gefäßbett in den interzellulären Raum aufgrund abnehmender Konzentrationen von Albumin als onkotisch wirksames Eiweiß (z. B. durch Synthesestörungen bei Lebererkrankungen). Für einen Blutdruckabfall können aber auch plötzliche oder schleichende Erhöhungen der Volumenkapazität der Gefäße verantwortlich sein. Plötzliche Gefäßerweiterungen resultieren beispielsweise aus akuten Massenausschüttungen von vasodilatatorischen Gewebshormonen, wie Histamin, Bradykinin, Kallikrein, Leukotrienen oder Prostaglandinen, die beim anaphylaktischen Schock auftreten. Schleichende Erweiterungen resultieren aus chronischem Druckanstieg in der Pfortader in Verbindung mit einer erhöhten Präsenz von Vasodilatatoren in den Arteriolen des Splanchnikusnetzes und führen über eine Vergrößerung der Volumenkapazität zum hepatorenalen Syndrom mit Ascitesbildung.

In jedem Fall besteht ein Missverhältnis zwischen Volumenkapazität und Füllvolumen, welches durch zwei Behandlungskonzeptionen beeinflusst werden kann.

An erster Stelle steht der Versuch der Erhöhung des intravasalen Volumenangebotes durch Verabreichung kristalloider oder kolloidaler Volumenersatzlösungen. Wird hierdurch der mittlere arterielle Druck nicht in den Bereich gebracht, der eine ausreichende Durchblutung aller Organe ermöglicht, kommen im zweiten Schritt "Pressoren" ("Vasokonstriktoren", z. B. Katecholamine) mit gefäßverengender Wirkung zum Einsatz. Eine solche Vasokonstriktion wird besonders bei Gefäßtonusverlust, wie er bei Lebererkrankungen und Sepsis vorkommt, angestrebt um die Volumenkapazität des Gefäßsystems wieder zu verringern.

Bei Erkrankungen, bei denen die akute oder chronische Vasodilatation durch erhöhte Spiegel von Vasodilatatoren hervorgerufen wird, ist die Aufrechterhaltung eines zufriedenstellenden Blutdruckes durch Infusionen zur Volumenerhöhung langfristig ohne Vasokonstriktoren nicht möglich. Solche intensivmedizinischen Problemfälle sind z. B. Leberversagen mit Druckabfall und hepatorenalen Funktionsstörungen (sekundärer Nierenausfall bei Leberversagen wegen Durchblutungsstörungen) oder die Sepsis. Beide Fälle sind mit hohen Sterblichkeiten verbunden und verursachen in der Medizin hohe Kosten.

Bisherige Lösungsansätze empfehlen die Verabreichung von Volumenersatzflüssigkeiten in Verbindung mit Vasokonstriktoren. Die Verweildauer herkömmlicher Volumenersatzflüssigkeiten in den Gefäßen ist jedoch eingeschränkt. Kristalloide (salzhaltige) Infusionslösungen diffundieren schnell in den interzellulären Raum. Volumenersatzlösungen mit Polymeren, z. B. Stärkelösungen (Hydroxyethylstärke, HAES) oder Gelatinelösungen (Gelafundin), sind auch nach längerer Zeit in den Gefäßen wirksam, da sie durch ihre wasserbindende Eigenschaft das Plasmawasser im Gefäßraum halten und somit das intravasale Volumen dauerhaft erhöhen können. Künstliche Polymere erzeugen aber Probleme aufgrund von Unverträglichkeiten.

Ein besonders geeignetes Volumenersatzmittel ist eine Lösung des natürlichen Kolloids Serumalbumin. Serumalbumin wird seit Jahrzehnten in der Medizin als Plasmaexpander eingesetzt und gilt als biologisch verträglichstes und damit am meisten bevorzugtes Volumenexpansionsmittel, obwohl es auch das teuerste ist.

Lösungen von humanem Serumalbumin für die Infusion sind kommerziell erhältlich. Allerdings müssen diese Lösungen für die Pasteurisierung und die Lagerung mit Stabilisatoren versetzt werden, um eine spontane Polymerisation des Albumins zu verhindern. Am häufigsten werden als Stabilisatoren N-Acetyltryptophan und Octansäure bzw. deren Natriumsalze allein oder in Kombination eingesetzt. Diese Stabilisatoren binden mit sehr hoher Affinität an das Albuminmolekül und besetzen und blockieren dabei für die biologische Funktion des Albumins wichtige Bindungsstellen.

Metaanalysen haben gezeigt, daß die Verwendung von Serumalbuminlösungen auf der Intensivstation im Vergleich zu anderen Plasmavolumenersatzlösungen mit einer erhöhten Mortalität einherging (Cochrane Metaanalyse im BMJ 1998;317, S. 235-240). Herkömmliche Albumininfusionslösungen scheinen daher mit Ausnahme von wenigen bestimmten Indikationen keinen klinischen Vorteil zu bringen. Inwieweit der Herstellungsprozeß (Fraktionierung mit anschließender Pasteurisierung und Stabilisierung) bei herkömmlichen Serumalbuminlösungen die theoretisch idealen Eigenschaften des Serumalbumins als Plasmaexpander nachteilig beeinflußt, ist derzeit nicht bekannt. In der Literatur wurde verschiedentlich die Vermutung geäußert, daß die Stabilisatoren N-Acetyltryptophan und Octansäure unter Umständen schädliche Nebenwirkungen haben könnten. Es ist daher wünschenswert, diese Stabilisatoren vor einer Verabreichung der Albuminlösung an einen Patienten zu entfernen, zumal diese auch die für wichtige Funktionen des Albumins erforderlichen Bindungsstellen mit hoher Affinität besetzen und blockieren. Allerdings stellt sich bei stabilisatorfreien Albuminlösungen wieder das oben erwähnte Problem der spontanen Polymerisation des Albumins und damit der schlechten Lagerungsfähigkeit solcher Lösungen.

Die biologisch wichtige Fähigkeit von humanem Serumalbumin, Liganden zu binden, ist Gegenstand vielfältiger Publikationen. Eine umfangreiche Darstellung findet sich u.a. in J. Peters jr., All about Albumin, Academic Press, San Diego, New York, Boston, London, Sydney, Tokyo Toronto, 1996, und in Pacifici GM, Viani A, Methods of Determining Plasma and Tissue Binding of Drugs, Clin Pharmacokinet, 1992, 23 (6): 449-468. Durch die enorme Vielfalt an Methoden zur Bestimmung der Albuminbindungskapazität sind die Ergebnisse jedoch schwer vergleichbar und eine Interpretation in Bezug auf ihre medizinische Relevanz praktisch nicht möglich.

Eine neue Methode zur Dokumentation des Bindungsverhaltens von Albumin stellt die Messung der Albuminbindungskapazität (ABiC) für Dansylsarkosin dar (Klammt S, Brinkmann B, Mitzner S, Munzert E, Loock J, Stange J, Emmrich J, Liebe S, Albumin Binding Capacity (ABiC) is reduced in commercially available Human Serum Albumin preparations with stabilizers, Zeitschrift für Gastroenterologie, Supplement 2001, 39: 24-27.) Diese Methode beruht auf der Messung des ultrafiltrierbaren Anteils des Testmarkers Dansylsarcosin unter definierten experimentellen Bedingungen und der Relation dieser Bindungskapazität zu einem Referenzalbumin.

In einem Vergleich zwischen gesunden Blutspendern und Patienten mit schwerer Lebererkrankung konnte eine signifikante Reduktion der Bindungskapazität des Serumalbumins nachgewiesen werden, die mit der verstärkten Besetzung der Bindungsplätze des Serumalbumins durch endogene Liganden als Folge von Leberentgiftungsstörungen bei den untersuchten Patienten erklärt wird. Bekannt ist, daß das Bindungsverhalten kommerziell verfügbarer Zubereitungen von humanem Serumalbumin gegenüber bestimmten Modellmarkern (z. B. Ibuprofen) gleichfalls dramatisch eingeschränkt ist.

Ebenso ist bekannt, daß bei einem ligandenfreien Albumin die Bindungskapazität für Dansylsarcosin von 100% bis auf ca. 60% gesenkt werden kann, wenn man schrittweise N-Acetyltryptophan bis zu einem molaren Verhältnis von 1:1 zugibt (gemessen anhand der ABiC nach Klammt et al., 2001).

In der technischen und medizinischen Literatur gibt es zahlreiche Publikationen zur Aufreinigung von Albumin aus Spenderplasma bzw. von biotechnologisch (rekombinant) hergestelltem Albumin. Diese Publikationen beziehen sich jedoch hauptsächlich auf die möglichst reine Präparation der Albuminfraktion und die Entfernung anderer Proteinbestandteile oder potentiell toxischer Komponenten aus dem Blutplasma bzw. im Fall rekombinanter Herstellung aus den Vektorsystemen.

Die Entfernung niedermolekularer Liganden, wie der Stabilisatoren in kommerziellen Serumalbuminlösungen, wurde bis 1967 entsprechend den Methoden von Goodman (Goodman DS, Science, 125, 1996, 1957), basierend auf der Extraktion mit einem Gemisch aus Isooktan und Essigsäure, bzw. von Williams (Williams EJ and Foster JF, J Am Chem Soc, 81, 965, 1959), basierend auf der spontanen Lipidschichtbildung im stark sauren Milieu, durchgeführt. Beide Methoden sind extrem zeitaufwendig und zur Herstellung therapeutischer Zubereitungen aufgrund potentieller Toxizität nicht geeignet. Nach diesen Methoden hergestellte Albuminlösungen weisen eine sehr schlechte Lagerungsstabilität auf.

Seit 1967 werden als Stabitisatoren in Albuminlösungen eingesetzte freie Fettsäuren, wie Octansäure, durch starke Ansäuerung und anschließende Aktivkohlebehandlung der Albuminlösung entfernt. Das Verfahren wurde erstmals publiziert von Chen et al., Journal of Biological Chemistry, Band 212, Nr. 2, 25. Januar, S. 173-181; 1967. Bei dem Verfahren wird die Albuminlösung in destilliertem Wasser mittels einer Säure (HCl) auf einen pH-Wert von 3 oder weniger angesäuert um eine Entfaltung des Albuminmoleküls durch die Auflösung von Wasserstoffbrückenbindungen zu ermöglichen und zusätzlich die Protonierung der entsprechenden Fettsäuren zu erreichen. Hierdurch wird die Bindung zwischen Albumin und Fettsäure so gelockert, daß die Fettsäure als kleines Molekül zur Aktivkohle diffundieren kann. Danach wird die Albuminlösung mit Aktivkohle gemischt und im Eisbad mittels eines Magnetrührers für 1 Stunde gerührt. Anschließend wird die Aktivkohle durch 20minütige Zentrifugetion der Mischung bei 20.200 x g abgetrennt. Nach dieser Methode lassen sich Abreicherungen verschiedener Fettsäuren erreichen. Dieses bis heute als Standard eingesetzte Verfahren zur Entfernung von Fettsäuren beruht auf detaillierten Untersuchungen verschiedener Bedingungen, wie unterschiedlichen pH-Werten und unterschiedlichen Masseverhältnissen von Aktivkohle zu Albumin, bei denen die o.g. Standardprozedur mit Abstand die erfolgreichste war. Die Ablösung der Stabilisatoren vom Albuminmolekül wurde somit nur durch eine Auflösung der Struktur des Albuminmoleküls und eine damit verbundene Herabsetzung der Bindungsaffinität im stark sauren Milieu erreicht. Eine wesentliche Abreicherung freier Fettsäuren aus humanem Serumalbumin bei höheren pH-Werten über 3 war nicht erfolgrelch.

Ein wesentlicher Nachteil des Verfahrens besteht in der strukturellen Veränderung des Albuminmoleküls durch die beträchtliche Ansäuerung im wässrigen Milieu. Damit werden nicht nur die schleifenbildenden Bindungen zwischen voneinander entfernten Aminosäuren gelöst, sondern auch die hydrophoben Bindungstaschen freigelegt, was zu einer vermehrten Adsorption des Albumins an die benutzte Aktivkohle führt. Chen et al. geben für ihre Methode Albuminverluste von 20% im Kohlepellet an. Das Verfahren ist für die primäre Herstellung kommerzieller therapeutischer Albuminlösungen ungeeignet, da durch die strukturellen Veränderungen des Albuminmoleküls eine spontane Polymerisierung des humanen Serumalbumins bei Lagerung ausgelöst wird.

Die US-A-2004/0217055 beschreibt die Entfernung von Fettsäuren aus einer wässrigen Lösung von rekombinant hergestelltem humanem Serumalbumin beispielsweise mittels Aktivkohle bei einem pH-Wert unter 3,5, wobei ein pH-Wert unter 3,0 als bevorzugt angegeben ist. Die zu entfernenden Fettsäuren stammen aus den Zelllinien, transgenen Tieren oder Pflanzen, aus denen das rekombinante Serumalbumin aufgereinigt wird, oder es handelt sich um Fettsäuren, die sich während des Extraktions- oder Reinigungsverfahrens an das Serumalbumin angeheftet haben. Auch das hierin offenbarte Verfahren ist mit den oben Beschriebenen Nachteilen behaftet.

Es ist daher eine Aufgabe der Erfindung ein Verfahren bereitzustellen, mit dem sich eine stabilisierte handelsübliche Albuminausgangslösung auf einfache, schnelle, kostengünstige und albuminschonende Weise, z. B. bettseitig unmittelbar vor der Verabreichung an einen Patienten, unter Entfernung eines großen Teils der Stabilisatoren und unter Erhöhung der Albuminbindungskapazität aufbereiten läßt, ohne das Albumin strukturell zu schädigen.

Insbesondere soll das Verfahren eine Erhöhung der Albuminbindungskapazität an der Bindungsstelle Sudlow II liefern, was für alle Anwendungen von Albumin in der intravenösen Volumenersatztherapie, aber auch bei extrakorporalen Entgiftungsverfahren, wie dem Plasmaaustausch gegen Albumin oder der extrakorporalen Albumindialyse, von Bedeutung für die Effektivität zur Immoblisierung endogener albuminaffiner Toxine ist.

Gelöst wird diese Aufgabe durch ein Verfahren zur Herstellung einer wässrigen Albuminlösung aus einer Albuminausgangslösung, welche Stabilisatormoleküle enthält, die in der Lage sind, Bindungsstellen des Albumins zu besetzen, wobei man bei dem Verfahren zur Erhöhung der Albuminbindungskapazität für andere Moleküle wenigstens einen Teil der Stabilisatormoleküle von dem Albumin der Albuminausgangslösung entfernt und von der Albuminausgangslösung abtrennt, indem man

a) die Albuminausgangslösung mit einem festen Adsorbermaterial in Kontakt bringt, dessen Affinität für wenigstens einen Teil, vorzugsweise für alle der eingesetzten Stabilisatormoleküle höher ist als die Affinität des Albumins für die entsprechenden Stabilisatormoleküle,

wobei das Adsorbermaterial ein partikuläres Aktivkohlematerial ist, welches in einer Säule oder einem Bett gepackt oder in eine Trägermatrix eingebettet ist, so dass zwischen den Partikeln des Adsorbermaterials flüssigkeitsdurchlässige Kanäle ausgebildet werden, wobei der mittlere Durchmesser der Kanäle, bezogen auf die gesamte Länge der zwischen den Partikeln des gesamten verwendeten Adsorbermaterials ausgebildeten Kanäle, mehr als 100 nm und weniger als 1000 $\mu$m beträgt und das Verfahren bei einem pH-Wert $\geq$ 5 durchgeführt wird und

b) die Albuminlösung von dem Adsorbermaterial abtrennt.

Besonders bevorzugt wird das Verfahren bei einem pH-Wert im Bereich von 5 bis 9, besser im Bereich von 6 bis 8 durchgeführt. Ganz besonders bevorzugt ist ein pH-Wert-Bereich von 6,9 bis 7,5.

Bei einem Viertel der gegenwärtigen medizinischen Anwendungen von humanem Serumalbumin (HSA) als Volumenersatzmittel (insges. ca. 200 Tonnen pro Jahr) spielen neben den kolloidosmotischen Eigenschaften auch die intakten Bindungseigenschaften für Toxine (z. B. Benzodiazepine) eine große Rolle, nämlich bei lebererkrankungsassoziierten Indikationen. Diese Eigenschaft ist jedoch in kommerziellen Zubereitungen durch Bindungsstellen okkupierende Stabilisatoren (N-Acetyltryptophan und Octanoat) eingeschränkt, was sich in einer verminderten Albuminbindungskapazität (ABiC) widerspiegelt. Das erfindungsgemäße Verfahren ermöglicht die anwendungsnahe Aufbereitung einer kommerziellen, stabilisierten Albuminlösung ohne aktive pH Manipulation. Die aufbereitete Lösung enthält Albumin, das eine erhöhte ABiC aufweist.

Dadurch werden die Eigenschaften des humanen Serumalbumins als Plasmaexpander klinisch so verbessert, daß die Bindungsleistung des Albumins der physiologischen Transport-fähigkeit des humanen Serumalbumins (HSA) angeglichen wird. Kreislauf- Nieren- und Hirnfunktion von Patienten werden positiv beeinflußt und das Kosten/Nutzen-Verhältnis wesentlich verbessert.

Die Erfindung beruht auf der überraschenden Erkenntnis, daß sich bei entsprechender Verfahrensführung und unter Verwendung des geeigneten Adsorbermaterials die zur Stabilisierung und Verhinderung der spontanen Polymerisation in kommerziellen Albuminlösung enthaltenen Stabilisatoren, insbesondere mittelkettige Fettsäuren (z. B. Octanoat), ohne eine drastische Absenkung des pH-Wertes einfach, schnell und ohne großen apparativen Aufwand in einem Ausmaß entfernen lassen, welches eine meßbare Erhöhung der Albuminbindungskapazität (ABiC) liefert. Das erfindungsgemäße Verfahren eignet sich daher besonders zur anwendungsnahen oder bettseitigen Aufbereitung kommerzieller stabilisierter Albuminlösungen, die unmittelbar nach der Aufbereitung einem Patienten verabreicht werden können. Das Verfahren hat weiterhin den Vorteil, daß das Albumin in der Lösung keinen Extrembedingungen, wie beispielsweise der drastischen Erniedrigung des pH-Wertes nach dem Stand der Technik ausgesetzt werden muß, um die Stabilisatoren von den Bindungsstellen des Albuminmoleküls durch Aufbrechen von Bindungen zwischen voneinander entfernten Aminosäuren der Albuminkette, welche die Schleifenbildung und Bindungsstelleneigenschaften bestimmen, zu entfernen. Es kommt bei dem erfindungsgemäßen Verfahren daher nicht zu der aus dem Stand der Technik als nachteilig bekannten strukturellen Veränderung des Albuminmoleküls.

DEFINITIONEN

**Albuminbindungskapazität (ABiC)**

Die Albuminbindungskapazität (ABiC) im Sinne dieser Erfindung wird nach der Methode von Klammt et al., 2001 bestimmt. In einer Albuminlösung wird zunächst die Albuminkonzentration durch Streuungs-Messung (Nephelometrie) bestimmt und die Lösung durch Verdünnen auf eine Albuminkonzentration von 150 $\mu$mol/l oder 300 $\mu$mol/l eingestellt. Anschließend wird ein Volumen der Albuminlösung mit einer definierten Konzentration eines für die Bindungsstelle II (Diazepam- Bindungsstelle) des Albumins spezifischen Fluoreszenzmarkers (Dansylsarcosin, Sigma Chemical) in einem equimolaren Verhältnis versetzt und für 20 min bei 25°C inkubiert. Nach der Inkubation wird ungebundener Fluoreszenzmarker durch Ultrafiltration (Centrisart I, Sartorius Göttingen; Ausschlußgröße: 20.000 Dalton) abgetrennt und die Menge an ungebundenem Fluoreszenzmarker in der abgetrennten Lösung durch Fluoreszenzspektrometrie bestimmt (Fluoroscan, Labsystems, Finnland; Anregung: 355 nm, Emission: 460 nm). Zur Fluoreszenzverstärkung wird der Lösung von ungebundenem Fluoreszenzmarker ligandenfreies Albumin (fettsäurefrei; von Sigma-Aldrich in Pulverform erhältlich) in einer Konzentration von 150 $\mu$mol/l oder 300 $\mu$mol/l zugesetzt. Parallel zur Probenalbuminlösung wird die gleiche Messung einer entsprechenden Lösung eines Referenzalbumins durchgeführt. Als Referenz wird gereinigtes und deligandisiertes humanes Serumalbumin (BiSeKo, Biotest Pharma GmbH, Dreieich, Deutschland) verwendet. Alternativ kann auch das Albumin aus einem Serumpool von mehr als 50 gesunden Blutspendern (nach den Kriterien des DRK) herangezogen werden. Die Albuminbindungskapazität (ABiC) wird nach der folgenden Formel berechnet:

$$\text{ABiC [\%]} = \frac{\text{Konz. ungebundener Fluoreszenzmarker (Referenzalbumin)} \times 100}{\text{Konz. ungebundener Fluoreszenzmarker (Probenalbumin)}}$$

Anmerkung: Die gemäß Klammt et al. gemessene und gemäß vorstehender Formel berechnete Albuminbindungskapazität (ABiC) gibt nicht die absolute Bindungskapazität des Albumins an allen seinen Bindungsstellen wieder, sondern vielmehr die bzgl. des Referenzalbumins relative Bindungskapazität für Liganden, die an die Sudlow II-Bindungsstelle (Diazepam- Bindungsstelle) binden. Sie kann daher auch Werte über 100% erreichen. Durch das spezielle Meßverfahren ist sie aber besonders gut dazu geeignet, auch kleinste Veränderungen der Albuminbindungskapazität zu erfassen, da sich der Marker besonders leicht aus der Bindung verdrängen läßt.

Handelsübliche Albuminlösungen, die mit N-Acetyltryptophan und/oder Octansäure bzw. deren Na-Salzen stabilisiert sind, besitzen üblicherweise eine Albuminbindungskapazität nach dem hierin verwendeten Bestimmungsverfahren von weniger als 60%. Die vorliegende Erfindung beruht auf der Anwendung eines Adsorptionsverfahrens mit einem Adsorber, der bei einem pH-Wert $\geq$ 5, vorzugsweise bei einem pH-Wert im Bereich von 5 bis 9, eine höhere Affinität für die verwendeten Stabilisatoren (z. B. Octansäure und/oder N-Acetyltryptophan) besitzt als das Albumin selbst. Mit dem erfindungsgemäßen Verfahren konnte eine Erhöhung der Albuminbindungskapazität in handelsüblichen stabilisierten Albuminlösungen ohne Ansäuerung bis auf über 100% (bezogen auf Referenzalbumin) innerhalb von 30 Minuten erreicht werden.

Ein wesentlicher Vorteil des erfindungsgemäßen Verfahrens ist, daß das Albumin dabei nicht durch extreme Bedingungen, wie starkes Ansäuern oder die Verwendung von denaturierenden Mitteln, strukturell stark verändert wird, sondern seine native Konformation im wesentlichen beibehält. Dadurch und durch die verbesserte Bindungskapazität nach Stabilisatorentfernung entfaltet es nach Infusion in einem Patienten eine deutlich höhere Aktivität als handelsübliche Albuminpräparationen. Ein weiterer Vorteil des erfindungsgemäßen Verfahrens ist die hohe Geschwindigkeit, mit der stabilisierte Albuminlösungen nach dem Verfahren von Stabilisatoren abgereichert werden können, und die geringen Anforderungen an den apparativen Aufwand und an die anschließende Aufbereitung der von Stabilisatoren abgereicherten Albuminlösung. So ist eine Renaturierung des Albumins nach der Entfernung der Stabilisatoren, wie beispielsweise durch spontane Rückbildung der inneren Schleifen des Albumins, was auch mit der Unsicherheit der spontanen Bildung entarteter oder polymerisierter Albuminmoleküle verbunden ist, nicht erforderlich. Üblicherweise wird die erfindungsgemäße Albuminlösung nach der Abreicherung lediglich durch einen Partikelfilter mit einer Porengröße über 65.000 Dalton geleitet, um eventuell vorhandene Grobteilchen zu entfernen. Danach ist die Lösung unmittelbar einsatzbereit für die Infusion in einen Patienten. Dies erlaubt eine anwendungsnahe (z. B. bettseitige) Durchführung des Verfahrens.

Da Albumin in der Regel an den Menschen verabreicht wird, z. B. als Plasmaexpander, wird erfindungsgemäß zweckmäßigerweise humanes Serumalbumin (HSA) verwendet. Obwohl sich das erfindungsgemäße Verfahren für die Abreicherung einer Vielzahl von Stabilisatoren oder anderen Liganden einsetzen läßt, ist es besonders geeignet für die Entfernung der Stabilisatormoleküle N-Acetyltryptophan und/oder Octansäure oder deren Anionen. Mit Vorteil kann das Verfahren für Liganden mit einem $K_a$-Wert (Assoziationskonstante) von mehr als $10^4$ eingesetzt werden.

In einer bevorzugten Ausführungsform des erfindungsgemäßen Verfahrens erfolgt das Inkontaktbringen der Albuminausgangslösung und des Adsorbermaterials in der oben genannten Stufe a) durch Hindurchleiten der Albuminausgangslösung durch eine das Adsorbermaterial enthaltende Säule (Chromatographiesäule).

In einer alternativen Ausführungsform erfolgt das Inkontaktbringen der Albuminausgangslösung und des Adsorbermaterials in der oben genannten Stufe a) durch Hindurchleiten der Albuminausgangslösung durch ein von dem Adsobermaterial gebildetes Bett. Besonders geeignet ist beispielsweise ein mittels eines sich langsam bewegenden Rührers oder durch Rütteln oder gegenläufige Strömungen sanft in Bewegung gehaltenes Wirbelbett. Hierdurch wird verhindert, daß die Strömungswege bzw. Strömungskanäle in einem dicht gepackten Bett aus Adsorbermaterial durch sehr kleine Partikel zugesetzt werden und der Durchfluß der Albuminlösung behindert oder ganz blockiert wird.

Wie oben bereits ausgeführt, erfolgt zweckmäßigerweise das Abtrennen der Albuminlösung von dem Adsorbermaterial in der oben genannten Stufe b) durch Filtration der Albuminlösung durch einen Partikelfilter, wobei der Partikelfilter so ausgewählt ist, daß er Albuminmoleküle hindurchtreten läßt und das feste Adsorbermaterial zurückhält.

In einer weiteren bevorzugten Ausführungsform des erfindungsgemäßen Verfahrens sind die Partikel des Adsorbermaterials an oder in einer Matrix gebunden. Geeignete Matrixmaterialien sind Stützgewebe (z. B. Polymerfasergewebe) oder offenporige Polymerschaumstrukturen (z. B. offenzellige Polyurethanschäume). In einer weiteren alternativen Ausführungsform können die Partikel des Adsorbermaterials auch einfach durch die Zumischung hochporöser Partikel als "Abstandshalter" in eine Form von Festbettreaktoren gebracht werden, die eine ausreichende Nähe der Adsorbermaterialpartikel zueinander und geeignete Kanalweiten liefert. Bei der Fixierung in hochporösen, offenzelligen Polymerschäumen ist auch die gleichzeitige oder anschließende Einarbeitung von Kanälen z. B. durch Bohrungsverfahren möglich. Vorteilhaft ist an diesen Ausführungsformen die mittels Gewebe bzw. Stützpolymer erreichbare "lockere" Packung, die den relativ hochviskösen Albuminlösungen wenig Perfusionswiderstände entgegensetzt. Außerdem kann dadurch der Filtrationsaufwand zur Rückhaltung von Mikropartikeln wesentlich geringer gestaltet werden als in den zuvor beschriebenen Ausführungsformen.

In einer weiteren bevorzugten Ausführungsform des erfindungsgemäßen Verfahrens werden die Stufen a) und b) mehrmals, vorzugsweise 2 bis 6 mal wiederholt, wobei jeweils die in Stufe b) gewonnene behandelte Albuminlösung in die Stufe a) zurückgeführt wird. Zur Verbesserung der Abreicherungsrate wird dabei vorzugsweise in der Stufe a) jeweils durch Entfernung von Stabilisatormolekülen regeneriertes und/oder frisches Adsorbermaterial eingesetzt. Dies kann durch Austausch des Adsorbermaterials in der dafür vorgesehenen Vorrichtung erfolgen, wobei besonders bevorzugt jedoch mehrere Adsorptionseinrichtungen mit Adsorbermaterial in Reihe hintereinander angeordnet sind, durch welche die Albuminlösung nacheinander hindurchgeleitet wird.

In einer weiterhin bevorzugten Ausführungsform des erfindungsgemäßen Verfahrens werden die Menge an eingesetztem Adsorbermaterial im Verhältnis zur Albuminkonzentration in der Albuminausgangslösung und/oder die Kontaktzeit zwischen Albuminausgangslösung und Adsorbermaterial in Stufe a) so ausgewählt, daß die Albuminbindungskapazität (ABiC) der hergestellten Albuminlösung, gemessen nach Klammt et al., wenigstens 60 %, vorzugsweise wenigstens 70 %, besonders bevorzugt wenigstens 80 %, ganz besonders bevorzugt wenigstens 90 % beträgt. Die hierfür erforderliche Menge an Adsorbermaterial und die Kontaktzeit werden in Abhängigkeit vom eingesetzten Ausgangsalbumin und dem gewählten apparativen Aufbau abhängen und lassen sich vom Fachmann auf dem Gebiet in Kenntnis der Erfindung einfach ermitteln.

In einer weiterhin bevorzugten Ausführungsform des erfindungsgemäßen Verfahrens werden die Menge an eingesetztem Adsorbermaterial im Verhältnis zur Albuminkonzentration in der Albuminausgangslösung und/oder die Kontaktzeit zwischen Albuminausgangslösung und Adsorbermaterial in Stufe a) so ausgewählt, daß die Konzentrationen an gebundenen und ungebundenen Stabilisatormolekülen, insbesondere an N-Acetyltryptophan und/oder Octansäure oder deren Anionen in der Albuminausgangslösung auf weniger als 70%, vorzugsweise weniger als 50%, besonders bevorzugt auf weniger als 30% ganz besonders bevorzugt auf weniger als 10% ihrer Ausgangskonzentrationen gesenkt werden. Die hierfür erforderliche Menge an Adsorbermaterial und die Kontaktzeit werden in Abhängigkeit vom eingesetzten Ausgangsalbumin und dem gewählten apparativen Aufbau abhängen und lassen sich vom Fachmann auf dem Gebiet in Kenntnis der Erfindung einfach ermitteln.

In einer weiterhin bevorzugten Ausführungsform des erfindungsgemäßen Verfahrens werden die Menge an eingesetztem Adsorbermaterial im Verhältnis zur Albuminkonzentration in der Albuminausgangslösung und/oder die Kontaktzeit zwischen Albuminausgangslösung und Adsorbermaterial in Stufe a) so ausgewählt, daß die Konzentrationen an gebundenen und ungebundenen Stabilisatormolekülen, insbesondere an N-Acetyltryptophan und/oder Octansäure oder deren Anionen in der Albuminausgangslösung auf weniger als 3.5 mol/mol Albumin, vorzugsweise weniger als 2.5 mol/mol Albumin, besonders bevorzugt auf weniger als 1.5 mol/mol Albumin ganz besonders bevorzugt auf weniger als 0.5 mol/mol Albumin ihrer Ausgangskonzentrationen gesenkt werden.

Das Adsorbermaterial zur Durchführung des erfindungsgemäßen Verfahrens ist ein partikuläres Material, welches in einer Säule oder einem Bett gepackt oder in eine Trägermatrix eingebettet ist, so daß zwischen den Partikeln des Adsorbermaterials flüssigkeitsdurchlässige Kanäle ausgebildet werden, wobei der mittlere Durchmesser der Kanäle, bezogen auf die gesamte Länge der zwischen den Partikeln des gesamten verwendeten Adsorbermaterials ausgebildeten Kanäle, mehr als 100 nm und weniger als 1000 $\mu$m, vorzugsweise weniger als 500 $\mu$m, besonders bevorzugt weniger als 300 $\mu$m, ganz besonders bevorzugt weniger als 200 $\mu$m, noch bevorzugter weniger als 100 $\mu$m beträgt.

Je geringer der Kanaldurchmesser ist, desto höher ist die Wahrscheinlichkeit bzw. die Häufigkeit, daß Albuminmoleküle mit den Wänden der von dem Adsorbermaterial gebildeten Kanäle in Kontakt kommen und desto höher ist die Abreicherungsgeschwindigkeit nach dem erfindungsgemäßen Verfahren. Allerdings dürfen die Kanäle auch nicht zu gering dimensioniert sein, da ansonsten die Strömungsgeschwindingkeit der Albuminlösung zu stark herabgesetzt wird. Es hat sich daher als ganz besonders zweckmäßig erwiesen, wenn der mittlere Durchmesser der Kanäle, bezogen auf die gesamte Länge der zwischen den Partikeln des gesamten verwendeten Adsorbermaterials ausgebildeten Kanäle, mehr als 100 nm und weniger als 60 $\mu$m und ganz besonders bevorzugt weniger als 10 $\mu$m beträgt.

In einer besonders bevorzugten Ausführungsform des erfindungsgemäßen Verfahrens ist die minimale Kontaktzeit zwischen Albuminausgangslösung und Adsorbermaterial in Stufe a) so ausgewählt, daß

$$\text{dm [}\mu\text{m] / 10 [}\mu\text{m/min]} \leq \text{Kontaktzeit [min]} \leq \text{dm [}\mu\text{m] / 0,1 [}\mu\text{m/min],}$$

wobei dm der mittlere Durchmesser der Kanäle, bezogen auf die gesamte Länge der zwischen den Partikeln des gesamten verwendeten Adsorbermaterials ausgebildeten Kanäle, ist.

Eine noch effizientere Abreicherung wird erreicht, wenn die minimale Kontaktzeit zwischen Albuminausgangslösung und Adsorbermaterial in Stufe a) so ausgewählt ist, daß

$$\text{dm [}\mu\text{m] / 4 [}\mu\text{m/min]} \leq \text{Kontaktzeit [min]} \leq \text{dm [}\mu\text{m] / 0,3 [}\mu\text{m/min],}$$

wobei dm der mittlere Durchmesser der Kanäle, bezogen auf die gesamte Länge der zwischen den Partikeln des gesamten verwendeten Adsorbermaterials ausgebildeten Kanäle, ist.

In dem erfindungsgemäßen Verfahren ist das Adsorbermaterial Aktivkohle. Die Aktivkohle wird zweckmäßigerweise als suspensionsfähiges Material oder auch als Schüttung, z. B. gepackt in eine Säule oder als ein Bett von Adsorbermaterial, eingesetzt. Wichtig ist, daß die Aktivkohlepartikel in der Schüttung zwischen den Partikeln Kanäle ausbilden, die einerseits ausreichend groß sind, so daß die Albuminlösung mit ausreichender Fließgeschwindigkeit durch das Adsorbermaterial hindurchströmen kann, und ausreichend eng sind, so daß die in der Albuminlösung enthaltenen Albuminmoleküle mit großer Häufigkeit während des Hindurchströmens mit den Aktivkohlepartikeln in direkten Oberflächenkontakt treten. Besonders bevorzugt ist die Schüttung der Aktivkohle so, daß die zwischen den Partikeln ausgebildeten Kanäle die oben genannten vorteilhaften Kanaldurchmesser aufweisen. Der mittlere Durchmesser der Kanäle zwischen den Aktivkohlepartikeln sollte daher, bezogen auf die gesamte Länge der zwischen den Aktivkohlepartikeln ausgebildeten Kanäle, mehr als 100 nm und weniger als 1000 $\mu$m, vorzugsweise weniger als 500 $\mu$m, besonders bevorzugt weniger als 300 $\mu$m, ganz besonders bevorzugt weniger als 200 $\mu$m, noch bevorzugter weniger als 100 $\mu$m beitragen,

Alternativ kann die Aktivkohle auch in eine feste, poröse Matrix, z. B. eine Polymermatrix, bestehend aus Zellulose-, Harzen- oder anderen Polymerfasern oder offenporigen Schäumen, eingebettet als Adsorbermaterial verwendet werden. Bei Einbettung der Aktivkohle in eine Matrix ist zu beachten, daß die Matrix ein Hindurchströmen der Albuminlösung zuläßt und die Matrix die Aktivkohlepartikel so trägt, daß sie mit der Albuminlösung in Kontakt treten können. Darüber hinaus sollte das Matrixmaterial eine Porosität aufweisen, daß die Poren darin Kanäle für das Hindurchströmen der Albuminlösung mit den oben genannten Kanaldurchmessern ausbilden.

Vorteilhaft ist die Verwendung einer Trägermatrix mit hydrophilen Eigenschaften, welche die Benetzung des Adsorbermaterials fördern. Eine solche Trägermatrix kann beispielsweise Zellulose oder andere hydrophile natürliche oder künstlich hergestellte Polymere umfassen.

Aktivkohle selbst ist ein poröses Material, das im Inneren der Aktivkohlepartikel Makroporen (> 25 nm), Mesoporen (1 - 25 nm) und Mikroporen (< 1 nm) aufweist, so daß die Aktivkohle eine sehr große innere Oberfläche besitzt. Die Größen dieser Poren werden bei Aktivkohle üblicherweise durch die Melassezahl (Makroporen), die Methylenblau-Adsorption (Mesoporen) und die Iodzahl (Mikroporen) angegeben. Die innere Oberfläche wird nach BET bestimmt und in m$^2$/g Aktivkohle angegeben. Aktivkohle ist allgemein als ein Adsorptionsmittel bekannt, welches Moleküle in seine Poren aufnimmt und darin festhält bzw. Substanzen durch Oberflächenbindungen immobilisiert. Aufgrund der hohen Porosität und inneren Oberfläche besitzt Aktivkohle eine sehr hohe Adsorptionskapazität, bezogen auf ihr Gewicht bzw. ihr äußeres Volumen. Dieses setzt allerdings voraus, daß die Moleküle in diese Poren hinein diffundieren können.

Der Stand der Technik bezüglich der Aufbereitung von handelsüblichen Albuminlösungen zeigt, daß allein die Verwendung von Aktivkohle als solche nicht ausreicht, um die sehr fest an das Albuminmolekül gebundenen Stabilisatoren einer stabilisierten Albuminlösung von dem Albuminmolekül zu entfernen, insbesondere nicht mit einer akzeptablen Geschwindigkeit. Dies bestätigen auch die bisher eingesetzten Verfahren zur Abreicherung von Stabilisatoren aus Albuminlösungen, in denen zwar Aktivkohle als Adsorptionsmittel in einer Aufschlämmung eingesetzt wird, jedoch ohne Erfolg bei höheren pH-Werten > 3. Erst nach starker Ansäuerung und einer damit einhergehenden strukturellen Veränderung oder Denaturierung des Albumins wird bei den bekannten Verfahren erreicht, daß sich die Stabilisatormoleküle von dem Albumin lösen und dann von der Aktivkohle gebunden werden können.

Die Erfinder haben nun herausgefunden, daß eine bestimmte Anordnung der Partikel des Adsorbermaterials, der Aktivkohle, im Sinne einer vorteilhaften Dimensionierung der Kanäle zwischen den Partikeln dazu führt, daß die Albuminmoleküle die fest daran gebundenen Stabilisatormoleküle leichter und schneller abgeben als dies bisher unter milden Bedingungen, wie bei pH-Werten > 3, bekannt war. Dabei sollten die Kanäle einen mittleren Durchmesser von mehr als 100 nm aufweisen, damit die Albuminmoleküle gut hindurchtreten können. Sie sollten also wesentlich größer sein als die für die Aktivkohleadsorption üblicher weise bestimmenden Meso- oder Mikroporen. Der mittlere Durchmesser der Kanäle sollte aber auch möglichst nicht mehr als 1000 μm betragen. Es wurde festgestellt, daß die Geschwindigkeit der Stabilisatorentfernung von den Albuminmolekülen umso größer ist, je geringer der mittlere Kanaldurchmesser ist, solange er über 100 nm liegt. Zweckmäßigerweise sind die Teilchen des Adsorbermaterials so angeordnet, daß die Kanäle jeweils wenigstens einen Eingang und einen Ausgang aufweisen, damit eintretende Albuminmoleküle nicht in diesen festgehalten werden, sondern aus den Kanälen auch wieder austreten können.

Darüber hinaus wurde herausgefunden, daß die Geschwindigkeit der Stabilisatorentfernung bei Verwendung von Aktivkohle als Adsorbermaterial weiter verbessert werden kann, wenn aus der Vielzahl erhältlicher und herstellbarer Aktivkohlen mit den unterschiedlichsten Porositäten und inneren Oberflächen als Adsorbermaterial solche ausgewählt werden, die eine Melassezahl (nach IUPAC) von 100 bis 400, vorzugsweise von 200 bis 300 aufweisen. Weiterhin vorteilhaft sind eine Methylenblau-Adsorption (nach IUPAC) von 1 bis 100 g/100 g Aktivkohle, vorzugsweise von 10 bis 30 g/100 g Aktivkohle, eine Iodzahl (nach IUPAC) von 500 bis 3000, vorzugsweise von 800 bis 1500, und/oder eine totale innere Oberfläche (BET) (nach IUPAC) von 100 bis 5000 m$^2$/g Aktivkohle, vorzugsweise von 800 bis 1400 m$^2$/g Aktivkohle.

Die Erfindung betrifft auch Adsorbermaterial mit den oben beschriebenen Merkmalen sowie dessen Verwendung für die Durchführung des erfindungsgemäßen Verfahrens. Des Weiteren betrifft die Erfindung auch die Verwendung einer wäßrigen Albuminlösung, hergestellt nach dem erfindungsgemäßen Verfahren, zur Herstellung eines Mittels für die Behandlung von Hypoalbuminaemie, eines Volumenersatzmittels bzw. Plasmaexpanders und/oder eines Mittels für die Verbesserung der Kreislauf-, Nieren- und/oder Hirnfunktion eines Patienten. Das Mittel ist auf die verstärkte Immobilisierung physiologisch wirkender Substanzen mit Affinität zu Albumin gerichtet.

Die Erfindung betrifft auch die Verwendung einer wäßrigen Albuminlösung, hergestellt nach dem erfindungsgemäßen Verfahren, zur Herstellung eines Mittels für die Blutreinigung, als Plasmaersatzmittel oder als Dialysat zur Albumindialyse. Insbesondere beim letztgenannten Einsatzgebiet ist eine erfindungsgemäß hergestellte Albuminlösung wesentlich preiswerter als vergleichbare Lösungen von ligandenarmen Albuminen, wie zum Beispiel rekombinantem humanem Serumalbumin.

Das erfindungsgemäße Verfahren bzw. eine nach dem Verfahren hergestellte Albuminlösung, die von Stabilisatoren abgereichert ist, ist für die Medizin von großem Nutzen. Patienten mit schweren Lebererkrankungen, hypotonen und hyperdynamen Kreislaufstörungen besitzen eine eingeschränkte Albuminbindungskapazität (ABiC), die durch heute verfügbare Standardalbuminpräparate nicht verbessert werden kann. Obwohl dieser Zusammenhang bisher experimentell noch nicht bewiesen worden ist, liegt die Theorie nahe, daß diese eingeschränkte Bindungskapazität eine Folge der endogenen Akkumulation albuminaffiner Toxine ist, die durch die geschädigte Leber nicht mehr physiologisch ausreichend abgebaut werden können. Versuche, diese eingeschränkte Albuminbindungskapazität durch kommerzielle Präparate von humanem Serumalbumin wieder herzustellen, scheitern an der herstellungsbedingten Überladung der Präparate mit Stabilisatoren. Diese Stabilisatoren sind jedoch für die Pasteurisierung der Albuminlösungen im Sinne der Virussicherheit und für die sichere Lagerung zur Verhinderung von Spontanpolymeren unabdingbar. Eine Entfernung dieser Stabilisatoren erforderte bisher eine extreme Ansäuerung und/oder war mit einem hohen Albuminverlust verbunden. Die Erfindung liefert daher ein Verfahren zur anwendungsnahen Herstellung einer Albuminlösung mit verbesserter Albuminbindungskapazität ohne aktive vorherige Ansäuerung.

Die erfindungsgemäß hergestellte Albuminlösung kann u.a. als ein Volumenersatzmittel eingesetzt werden mit einem von Stabilisatoren befreiten Albumin mit hohem Bindungsvermögen für vasoaktive Substanzen und Toxine mit einer Affinität für die Diazepambindungsstelle des Albumins (sogenannte Sudlow Bindungsstelle II). Das erfindungsgemäße Verfahren ist kostengünstig und apparativ mit geringem Aufwand durchzuführen, so daß es beispielsweise bettseitig kurz vor der Infusion der Lösung in einen Patienten bequem durchgeführt werden kann.

Durch die erfindungsgemäß verbesserte Bindungskapazität des Albumins wirkt die Albuminlösung als Volumenersatz nicht nur, wie man es bisher annahm, durch eine erhöhte kolloidosmotische, also wasserbindende Wirkung im Gefäß, sondern auch durch eine aktive Immobilisierung vasodilatatorischer und anderer toxischer Substanzen. Dies führt zu einer Abnahme der Vasodilatation und damit zu einem überadditiven Effekt des Volumenersatzes auf den Füllungszustand des Gefäßbettes. Letzterer wird am einfachsten durch den diastolischen Blutdruck ermittelt, der erheblichen Einfluß auf den arteriellen Mitteldruck und damit die Perfusion des Gefäßsystems hat.

Des Weiteren kann die erfindungsgemäß hergestellte Albuminlösung mit Vorteil zur besseren Bindung von Liganden in der Albumindialyse eingesetzt werden.

Das erfindungsgemäße Verfahren hat den Vorteil, daß die Abreicherung von Stabilisatoren aus einer Albuminlösung mit hoher Geschwindigkeit erfolgt, d. h. daß sie in relativ kurzer Zeit durchgeführt werden kann. So kann die Behandlung einer handelsüblichen Albuminlösung innerhalb von 10 bis 30 min durchgeführt und eine deutliche Erhöhung der Albuminbindungskapazität erreicht werden. Das Verfahren eignet sich daher unter anderem ideal für eine bettseitige Aufbereitung handelsüblicher stabilisierter Albuminlösungen, obwohl die Erfindung nicht darauf beschränkt ist. Auch eine Abreicherung in den Apotheken von Kliniken oder anderen Einrichtungen oder Unternehmen ist möglich.

Nach dem erfindungsgemäßen Verfahren wird eine Abreicherung einer handelsüblichen mit Octansäure (Ocatoat) und/oder N-Acetyltryptophan (N-Acetyltryptophanat) stabilisierten Albuminlösung auf eine jeweilige Konzentration dieser Stabilisatoren von unter 5 mol/mol Albumin, bevorzugt unter1 mol/mol Albumin, besonders bevorzugt unter 0,2 mol/mol Albumin erreicht.

Die Erfindung wird nun anhand von Beispielen weiter erläutert.

<u>BEISPIELE</u>

<u>Beispiel 1: Schüttung von Adsorbermaterial in einer gepackten Säule</u>

In einem Experiment zum Vergleich der Effizienz hinsichtlich der Abreicherung von Stabilisatormolekülen aus stabilisierter Albuminlösung wurden verschiedene Adsorbermaterialpräparationen hergestellt und getestet.

Für zwei erfindungsgemäße Präparationen von Adsorbermaterial wurde eine definierte Aktivkohle Norit GAC 830 (Norit) durch Vermahlen in einem industrieüblichen Grinder zu unterschiedlichen Partikelgrößen verarbeitet. Die Partikelgrößen der vermahlenen Produkte wurden anschließend mikroskopisch sowie durch handelsübliche Partikelanalysierer vermessen. Für einen ersten erfindungsgemäßen Ansatz (A1) wurde Norit GAC 830 zu einer Partikelgröße von 1 mm (D50-Wert) und für einen zweiten Ansatz (A2) wurde Norit GAC 830 zu einer Partikelgröße von 0,1 mm (D50 Wert) vermahlen. D50 entspricht der 50. Percentile (siehe auch Seite 22). Für einen Vergleichsansatz (V) wurde eine Aktivkohle Norit ROX Stäbchenkohle (Norit) verwendet.

Jeweils 100g der Aktivkohlematerialien wurden in mit einem Siebboden versehene Säulen mit einem Durchmesser von 6 cm und einer Höhe von 10 cm gefüllt und gewässert. Die Adsorbermaterialien in den Säulen wurden jeweils mit 20 g handelsüblichem Albumin in 330 ml Kochsalzlösung (ZLB Behring, pH 7.2) rezirkulierend mit einer Rate von 170 ml/min perfundiert. Zum Zeitpunkt Null sowie nach 20, 30, 60 und 120 min wurden die Konzentrationen von Octanoat, N-Acetyltryptophanat und Albumin sowie die Albuminbindungskapazität (nach Klammt et al. 2001) bestimmt. Für den Vergleichsansatz wurden darüber hinaus die Werte auch noch zu den Zeitpunkten 240, 1440 und 2880 min bestimmt. Die Ergebnisse sind in Tabelle 1 wiedergegeben.

Das Material Norit ROX Stäbchenkohle bildet bei Füllung in eine Säule aufgrund der Stäbchenform zwischen den Partikeln sehr weite Zwischenräume bzw. Kanäle aus, deren Durchmesser zum Teil 1 mm überschreiten, was für den Perfusionsdruck, insbesondere bei hohen Geschwindigkeiten von 100 bis 250 ml/min von Vorteil ist. Der Vergleich mit den erfindungsgemäßen Ansätzen A1 und A2 mit vermahlener Aktivkohle zeigt jedoch, daß dieses Adsorbermaterial mit den entsprechend großen Kanalweiten für eine Anhebung der Albuminbindungskapazität in kurzer Zeit praktisch nicht geeignet ist.

Für die Schüttungen der vermahlenen Aktivkohlematerialien der Ansätze A1 und A2 in den Säulen lassen sich die Kanaldurchmesser entsprechend der folgenden Formel (I) in guter Näherung angeben:

$$R_K = [R_P^2 + (R_P {}^*0.57735)^2]^{0.5} - R_P \qquad\qquad (I)$$

wobei $R_K$ der mittlere Kanalradius zwischen den Partikeln ist und $R_P$ der mittlere Radius der Partikel selbst ist.

Für die vermahlenen Partikel gemäß Ansatz A1 mit einem mittleren Durchmesser von 1 mm, d. h. einem Radius

$R_P$ von 500 $\mu$m ergibt sich ein mittlerer Kanalradius $R_K$ von 77 $\mu$m, d. h. eine mittlere Kanalweite (auch mittlerer Durchmesser) von etwa 150 $\mu$m. Für die vermahlenen Partikel gemäß Ansatz A2 mit einem mittleren Durchmesser von 0,1 mm, d. h. einem Radius $R_P$ von 50 $\mu$m ergibt sich ein mittlerer Kanalradius $R_K$ von 7,7 $\mu$m, d. h. eine mittlere Kanalweite (auch mittlerer Durchmesser) von etwa 15 $\mu$m.

Die Ergebnisse dieser Berechnung der Kanalweiten auf der Basis der o.g. Formel wurde auch durch Fixierung von Proben der Säulenmaterialien in Harz und anschließende mikroskopische Untersuchung von Dünnschnitten bestätigt.

**Tabelle 1**

| Kontaktzeit | Ansatz | mittlere Kanalweite [$\mu$m] | Octanoat/ Albumin [mol/mol] | NAC/ Albumin [mol/mol] | ABiC [%] |
|---|---|---|---|---|---|
| 0 | V | 1000 | 5,2 | 5,2 | 45 |
| | A1 | 150 | 5,2 | 5,2 | 45 |
| | A2 | 15 | 5,2 | 5,2 | 45 |
| 20 | V | 1000 | 1,05 | <0,2 | 48 |
| | A1 | 150 | 0,9 | <0,2 | 63 |
| | A2 | 15 | 0,4 | <0,2 | 82 |
| 30 | V | 1000 | 0,96 | <0,2 | 51 |
| | A1 | 150 | 0,9 | <0,2 | 65 |
| | A2 | 15 | 0,3 | <0,2 | 95 |
| 60 | V | 1000 | 0,69 | <0,2 | 56 |
| | A1 | 150 | 0,4 | <0,2 | 80 |
| | A2 | 15 | 0,2 | <0,2 | 100 |
| 120 | V | 1000 | 0,36 | <0,2 | 75 |
| | A1 | 150 | 0,2 | <0,2 | 95 |
| | A2 | 15 | 0,1 | <0,2 | 110 |
| 240 | V | 1000 | 0,23 | <0,2 | 81 |
| 1440 | V | 1000 | 0,07 | <0,2 | 96 |
| 2880 | V | 1000 | 0,05 | <0,2 | 103 |

Die Ergebnisse mit Norit ROX bestätigen die oben beschriebene Tatsache, daß unter physiologischen Bedingungen, insbesondere bei neutralem pH-Wert, eine wesentliche Abreicherung der Stabilisatoren unter den bislang angewendeten experimentellen Bedingungen, d. h. der Verwendung von Aktivkohleschüttungen oder -suspensionen mit großen Kanalweiten, in 60 min nicht möglich ist, zumindest nicht in einem Ausmaß, welches die Albuminbindungskapazität wesentlich erhöht.

Überraschenderweise konnte allerdings eine Verlängerung der Kontaktzeit gemäß den in dieser Anmeldung gemachten Angaben selbst bei dieser Kohle eine Abreicherung von Octanoat ermöglichen, die zu einem Anstieg der Albuminbindungskapazität führt. Entsprechend den Richtlinien zur Abschätzung der minimal nötigen Kontaktzeit in Abhängigkeit von den Kanalweiten sollte bei 1000 $\mu$m mittlerer Weite (Dm) die Kontaktzeit über 250 min ausgedehnt werden. Die Experimente zeigen, daß bei 240 min die ABiC nur gering auf 80% angestiegen ist, akzeptable Werte auf über 90% erst nach einem Tag (1440 min) gemessen werden konnten.

Bei einer Untersuchung der Oberflächenstruktur der getesteten Aktivkohlen war festgestellt worden, daß nur ein verschwindend geringer Anteil der Albuminmoleküle tatsächlich einen direkten Kontakt mit der Aktivkohlenoberfläche haben konnte, da einerseits lediglich die Makroporen überhaupt einen gewissen Zutritt des Albuminmoleküls erlauben, während Mesoporen in der Überzahl bereits zu klein für eine Albuminpassage sind. Die Makroporen machen jedoch lediglich einen verschwindend geringen Anteil der Porenverteilung aus und verzweigen sich sehr schnell und unmittelbar unter der äußeren Oberfläche zu Mesoporen, die für das Albuminmolekül nicht mehr passierbar sind. Wo Makroporen den Zutritt des Albuminmoleküls noch ermöglichen, werden zudem die Albuminmoleküle zu einem großen Teil in den Poren festgehalten, was besonders bei makroporösen Kohlen zu einem beträchtlichen Albuminverlust führen kann.

Die Versuchsansätze A1 und A2 zeigen, daß dieses Problem durch die Schaffung enger mittlerer Kanalweiten, beispielsweise durch Verwendung von Partikeln mit geringerem mittleren Durchmesser in einer Schüttung, gelöst werden kann. Die Ergebnisse bestätigen damit weiterhin, daß die Anordnung des Adsorbermaterials eine wesentliche Rolle für die Effektivität der Entfernung von Stabilisatoren spielt, die durch die Wahl des Adsorbers allein nicht automatisch erreicht wird.

In dem erfindungsgemäßen Verfahren wird daher ein Adsorbermaterial eingesetzt, das bezüglich des Albuminmoleküls dimensionierte Kanäle aufweist. Hierbei handelt es sich um Kanäle, die in ihrer gesamten Länge von dem Albuminmolekül passierbar sind und die so bemessen sind, daß die Albuminmoleküle sehr häufigen und engen Kontakt mit der Oberfläche des Adsorbermaterials bekommen. Die Kanäle sind durch ihre mittlere lichte Weite bzw. ihren mittleren Durchmesser charakterisiert.

Beispiel 2: Suspensionen von Aktivkohle- Partikeln in Wirbelbettreaktoren:

Um zu zeigen, daß nicht die Vergrößerung der äußeren Oberfläche des Aktivkohlematerials durch den Mahlprozeß, sondern die Einstellung der Kanäle zwischen den Partikeln durch das Material mit optimierten mittleren Kanaldurchmessern für den erfindungsgemäßen Erfolg entscheidend ist, wurden in einem weiteren Experiment Adsorbermaterialien mit gleichen äußeren Oberflächen ausschließlich unter Variation der Kanalweiten untersucht. Der geeignete Versuchsaufbau hierfür ist das sogenannte Wirbelbett, in dem das Adsorbermaterial fein partikuliert durch Rühren, Schütteln oder auch konvektive bzw. turbulente Strömungen in Suspension gehalten wird.

Im Wirbelbett stellen die Zwischenräume zwischen den in der Suspension gleichverteilten Partikeln die für den Albuminflußverlauf ausgebildeten Kanäle dar. Die Kanaldurchmesser im Wirbelbett lassen sich entsprechend der folgenden Formel (II) in guter Näherung angeben:

$$D_K = [V_{WB}^{0.33} - (n^{0.33} * D_P)]/ n^{0.33} \qquad (II)$$

wobei $D_K$ der mittlere Kanaldurchmesser zwischen den Partikeln, $D_P$ der mittlere Durchmesser der Partikel selbst, n die Partikelanzahl und $V_{WB}$ das Volumen des Wirbelbetts ist.

Das einzustellende Volumen des Wirbelbetts für einen gewünschten mittleren Kanaldurchmesser ergibt sich nach Auflösung der Formel (II) nach dem Volumen gemäß folgender Formel (Ia):

$$V_{WB} = [(n^{0.33} * D_P) + (n^{0.33} * D_K)]^3 \qquad (IIa)$$

Zur Einstellung des Kanaldurchmessers sind somit nur das Volumen des Wirbelbettes, die Partikelzahl und die Partikelgröße so aufeinander abzustimmen, daß der gewünschte Kanaldurchmesser erhalten wird. Der mittlere Partikeldurchmesser $D_P$ und auch die zur Abschätzung der Partikelzahl pro Einwaage notwendige Schüttdichte werden entweder vom Hersteller angegeben oder lassen sich nach einfachen Standardverfahren bestimmen.

Die Partikelanzahl n in einer Trockenschüttung läßt sich in für praktische Zwecke akzeptabler Näherung über die Schüttdichte (gestampft) und die Partikelgröße nach der folgenden Formel (III) berechnen:

$$n = [V_{TS}^{0.33}/(0.86 * D_P)]^3 \qquad (III)$$

wobei n die Partikelanzahl in einem trockenen Schüttvoiumen, $V_{TS}$ das Volumen der gestampften Trockenschüttung und $D_P$ der mittlere Partikeldurchmesser ist. Sofern für ein bestimmtes Adsorbermaterial die Trockenschüttdichte angegeben ist, läßt sich das Volumen $V_{TS}$ auch durch Teilen der Einwaage durch die Trockenschüttdichte errechnen.

Da in der Praxis die Partikel nicht immer ideal sphärisch und oft auch nicht immer gleich bleibend groß sind, muß bei Partikelmengen mit einer breiten Größenverteilung in der Praxis davon ausgegangen werden, daß die Verteilung der Zwischenräume in einer Suspension im Adsorberwirbelbett von der Größenverteilung der Adsorberpartikel abhängig ist. In der Regel werden besonders bei pulverisierten Adsorbern (z.B. bei Norit C Extra USP) die Kennzahlen der Partikelverteilung angegeben, d. h. die Percentilen der Größenverteilung, z. B. D10 und D90. Mittels der D10- und D90-Werte kann eine Größenverteilungsspannbreite beschrieben werden, die etwa 80% der Partikel umfaßt. Damit läßt sich praktikabel eingrenzen, auf welches Volumen man eine definierte Einwaage bzw. ein Trockenschüttvolumen im wäßrigen Wirbelbett verteilen darf, um die gewünschten Kanaldurchmesser in guter Näherung zu erhalten.

In diesem Fall sind die Berechungen des Wirbelbettvolumens unter Anwendung des D10-Wertes anstelle von $D_P$ und dann des D90-Wertes anstelle von $D_P$ durchzuführen, um die jeweiligen oberen und unteren Grenzen des Wirbelbettvolumens zu ermitteln, in denen der erfindungsgemäße Abstand zwischen den Adsorberpartikeln (Kanalweite) erfindungsgemäß optimiert ist. Die Berechnung auf der Basis des D90-Wertes wird zu einer Extremwertvariante führen, bei der unter Einhaltung der erfindungsgemäßen Parameter eine Deligandisierung mit effektiver Anhebung der Albuminbindungskapazität (ABiC) mit Sicherheit möglich ist.

Der Einfluß der Kanalweite wurde folgendermaßen belegt. Ein Aktivkohle-Albumin-Gemisch aus 1 g Aktivkohle / 1 g Albumin (Aktivkohle: Norit-C Extra USP von NORIT Nederland BV, Niederlande; handelsübliches Albumin, stabilisiert mit 5.2 mmol Octanoat und 5.2 mmol N-Acetyltryptophanat pro mmol Albumin) wurde in verschiedenen Volumina zum Wirbelbett in NaCl-Lösung aufgefüllt und 30 min bei Raumtemperatur gerührt. Nach dem Behandlungszeitraum wurden die Aktivkohlepartikel durch Zentrifugation von der Albuminlösung abgetrennt. Anschließend wurden die Albumin-, Octanoat- und N-Acetyltryptophanatkonzentrationen sowie die ABiC gemessen und im Verhältnis zu den errechneten Kanalweiten betrachtet. Die Ergebnisse sind in Tabelle 2 wiedergegeben.

**Tabelle 2**

| Test | Einwaage [g] | n50 | n10 | n90 | Wirbelbettvolumen [ml] | Dk50 [μm] | Dk10 [μm] | Dk90 [μm] | Oct /Alb | NAC /Alb | ABiC [%] |
|---|---|---|---|---|---|---|---|---|---|---|---|
| 1 | 0.5 | $3 \times 10^8$ | $2.94 \times 10^{10}$ | $6.7 \times 10^6$ | 3.25 | **5.2** | 1.2 | 17.2 | <0.2 | <0.2 | 110 |
| 2 | 0.5 | $1.5 \times 10^8$ | $1.48 \times 10^{10}$ | $3.36 \times 10^6$ | 7.5 | **12.3** | 2.8 | 42.4 | 0.20 | <0.2 | 83.2 |
| 3 | 0.5 | $1.5 \times 10^8$ | $1.48 \times 10^{10}$ | $3.36 \times 10^6$ | 10 | **15.9** | 3.6 | 54.8 | 0.22 | <0.2 | 79.9 |
| 4 | 0.5 | $1.5 \times 10^8$ | $1.48 \times 10^{10}$ | $3.36 \times 10^6$ | 12.5 | **18.8** | 4.2 | 65.3 | 0.27 | <0.2 | 70.3 |
| 5 | 0.5 | $1.5 \times 10^8$ | $1.48 \times 10^{10}$ | $3.36 \times 10^6$ | 22.5 | **27.8** | 6.2 | 96.8 | 0.43 | <0.2 | 67.5 |
| 6 | 0.5 | $1.5 \times 10^8$ | $1.48 \times 10^{10}$ | $3.36 \times 10^6$ | 32.5 | **34.3** | 7.6 | 119.8 | 0.58 | <0.2 | 63.3 |

- n50, n10, n90 = Teilchenzahlen, berechnet nach Formel (III) unter Verwendung der Percentilen D50, D10 bzw. D90 als mittlere Partikeldurchmesser.
- Dk50, Dk10, Dk90 = mittlere Kanaldurchmesser, berechnet nach Formel (II) unter Verwendung der Percentilen D50, D10 bzw. D90 als mittlere Partikeldurchmesser.
- Oct/Alb = molares Verhältnis von Octanoat zu Albumin.
- NAC/Alb = molares Verhältnis von N-Acetyltryptophanat zu Albumin.

Die Ergebnisse zeigen deutlich, daß eine Zunahme des mittleren Kanaldurchmessers (DK50) im Wirbelbett, welcher nach der oben angegebenen Formel (II) bei konstanter Partikelart und -anzahl direkt mit der Zunahme des Wirbelbettvolumens im Zusammenhang steht, unabhängig von der äußeren Oberfläche des Adsorbermaterials zu einer Abnahme der Effektivität bei der Abreicherung von Octanoat und N-Acetyltryptophan und der resultierenden Albuminbindungskapazität führt.

Gleichermaßen läßt sich der Tabelle entnehmen, daß unter Anwendung der Regel für eine erfindungsgemäß bevorzugte Ausführungsform

$$\text{dm [µm] / 4 [µm/min]} \leq \text{Kontaktzeit [min]} \leq \text{dm [µm] / 0,3 [µm/min]},$$

eine optimale Abreicherung innerhalb von 30 min dann erreicht wird, wenn die Kanalweite so gewählt wird, daß die obere Grenze der minimal nötigen Kontaktzeit unterhalb von 30 Minuten liegt (Test 1). In diesem Fall läge die obere Grenze der minimal notwendigen Kontaktzeit, bezogen auf den mittleren Kanaldurchmesser von 5.2 $\mu$m, bei 17.3 min, weshalb eine maximale Abreicherung von NAC und Octanoat erfolgte und die ABiC einen Wert von 110% erreichte. In den Tests 2 bis 6 wurde dieser obere Wert der minimal nötigen Kontaktzeit nicht überschritten. Daher wurden zwar sehr gute Abreicherungen und verbesserte ABiC beobachtet, jedoch wurde der optimale Wert von Test 1 nicht erreicht.

Die Ausführungsbeispiele 1 und 2 zeigen, daß die für eine effektive Stabilisatorabreicherung erforderliche Kontaktzeit zwischen Albuminlösung und Adsorbermaterial stark von dem mittleren Durchmesser der Kanäle in dem Adsorbermaterial abhängt. Durch Anpassung des mittleren Durchmessers der Kanäle des Adsorbermaterials lassen sich somit auch die erforderlichen Kontaktzeiten und damit auch beispielsweise bei einer mit Adsorbermaterial gepackten Säule die Durchflußgeschwindigkeit der Albuminlösung beeinflussen.

Beispiel 3: Fixierung von Adsorbern in Geweben, offenzelligen Schäumen oder Mischschüttungen

In der nachfolgend beschriebenen Ausführung wurden die erfindungsgemäßen Abstände zwischen den Adsorberpartikeln, d. h. die Kanalweiten in einem perfundierbaren Netzwerk "fixiert".

Die Partikel des Adsorbermaterials, z.B Aktivkohle, können dabei durch Stützgewebe (z. B. Polymerfasergewebe), offenporige Polymerschaumstrukturen (z. B. offenzellige Polyurethanschäume) oder aber auch einfach durch die Zumischung hochporöser Partikel als "Abstandshalter" in eine Form von Festbettreaktoren gebracht werden, die eine ausreichende Nähe der Adsorbermaterialpartikel zueinander und somit die erfindungsgemäß geeigneten Kanalweiten liefert. Bei der Fixierung in hochporösen, offenzelligen Polymerschäumen ist auch die gleichzeitige oder anschließende Einarbeitung von Kanälen z. B. durch Bohrungsverfahren möglich. Auch diese Kanalweiten entsprechen den Regeln, die die Relation zwischen der Kanalweite und der minimal nötigen Kontaktzeit erfindungsgemäß bestimmen.

Vorteilhaft ist an diesen Ausführungsformen die mittels Gewebe bzw. Stützpolymer erreichbare "lockere" Pak-kung, die den relativ hochviskösen Albuminlösungen wenig Perfusionswiderstände entgegensetzt. Außerdem kann dadurch der Filtrationsaufwand zur Rückhaltung von Mikropartikeln wesentlich geringer gestaltet werden als in den zuvor beschriebenen Ausführungsbeispielen.

Auch bei Fixierungen von Adsorbern in Geweben, offenzelligen Schäumen oder Mischschüttungen läßt sich der mittlere Kanaldurchmesser entsprechend der folgenden Formel (IV) in guter Näherung angeben:

$$D_K = [V^{0.33} - (n^{0.33} * D_P)]/ n^{0.33} \qquad\qquad (IV)$$

wobei $D_K$ der mittlere Kanaldurchmesser zwischen den Partikeln, $D_P$ der mittlere Durchmesser der Partikel selbst, n die Partikelanzahl und V das Volumen des Gewebes, der Mischschüttung oder des Schaums im Endzustand ist. Ein gewünschter Kanaldurchmesser kann nach Umstellung der Formel IV bei bekannter Partikelanzahl und bekanntem Partikeldurchmesser durch Errechnung des geeigneten Volumens eingestellt werden:

$$V = [(n^{0.33} * D_P) + (n^{0.33} * D_K)]^3 \qquad (IVa)$$

Die Berechnungsgrundlagen für die zweckmäßige Kombination zwischen AdsorberEinwaage und Endvolumen entsprechen dabei allen Berechnungsgrundlagen gemäß Beispiel 2.

In einem Experiment wurden 2g Aktivkohle (Norit-C Extra USP von NORIT Nederland BV, Niederlande) mit einer mittleren Partikelgröße von 23 $\mu$m (D50-Wert) (D10 = 5 $\mu$m, D90 = 82 $\mu$m) in einer wäßrigen Suspension/Lösung aus einem Faserpolymer (in diesem Fall Cellulose) und einem Polymer mit der Tendenz zur Vernetzung (z.B. Harze, Polyurethane, Polyacrylmethaacryllate usw.) vermischt, wobei eine Kanalweite von 3,6 $\mu$m eingestellt wurde, um eine zuverlässige Deligandisierung innerhalb von 12 min Kontaktzeit zu erreichen. Nach der erfindungsgemäßen Formel

$$\text{dm [µm]} / 4 \text{ [µm/min]} \leq \text{Kontaktzeit [min]} \leq \text{dm [µm]} / 0{,}3 \text{ [µm/min]},$$

beträgt die obere Grenze der minimal einzuhaltenden Kontaktzeit somit 12 min. Unter Anwendung der Formel (IVa) ergab sich für eine Einwaage von 2 g ein einzustellendes Volumen von 12,5 ml, was dem abschließenden Gesamtvolumen der Mischung entsprach. Die Mischung wurde auf ein engmaschiges Netz mit einer Oberfläche von ca. 25 cm$^2$ gegeben, dessen Poren klein genug waren, um Adsorberpartikel und vernetzte Stützpolymere nicht passieren zu lassen (z.B. 5 $\mu$m Maschenweite). Die Mischung wurde auf dem Netz so verteilt, daß nach Entwässerung durch einen Druckgradienten (1 atm) eine Trockendicke von 5 mm erreicht wurde. Das so hergestellte Adsorbermaterial hatte damit ein Endvolumen von insgesamt 12,5 ml. Der mittlere Kanaldurchmesser $D_K$, berechnet nach der oben genannten Formel (IV) und ausgehend von der Partikelgröße D50 = 23 $\mu$m, betrug 3.6 $\mu$m.

10 ml einer 20%-igen, handelsüblich stabilisierten Albuminlösung wurden mit einer Rate von 1 ml/min durch das Adsorbermaterial senkrecht zur Oberfläche des Netzes in einer handelsüblichen Filtereinspanneinrichtung in der selben Flußrichtung, wie der Druckgradient beim Trocknen aufgebaut war, hindurchgeleitet. Die Albuminbindungskapazität der so behandelten Albuminlösung wurde nach diesem Verfahren durch Entfernung von Stabilisatoren innerhalb von 10 min von 45% auf über 100% gesteigert. Das Verhältnis von Octanoat und N-Acetyltryptophanat zu Albumin lag jeweils unterhalb von 0.2 mmol/mmol.

Beispiel 4: Klinischer Einsatz

In klinischen Versuchen wurden die Effekte von erfindungsgemäß hergestellten Albuminlösungen mit erhöhter Albuminbindungskapazität untersucht. Eine prospektiv randomisiert gewählte Population von 30 Patienten mit Leberversagen bei chronisch ethyltoxischer Zirrhose und überlagerter C2-Hepatitis mit einem Bilirubinspiegel über 20 mg/dl und einer eingeschränkten Proteinsynthese (INR erhöht) mit hypotoner und hyperdynamer Kreislaufsituation wurde in zwei Gruppen aufgeteilt. Eine Gruppe wurde mit der erfindungsgemäß hergestellten Albuminlösung mit erhöhter Albuminbindungskapazität behandelt und die Kontrollgruppe nicht. Kreislaufparameter und Endorganfunktionen der Niere und des Gehirns wurden bei allen Patienten während der Versuchsdauer und des Beobachtungszeitraums von 2 Wochen regelmäßig überwacht.

Die Ergebnisse sind in den anhängenden Figuren gezeigt.

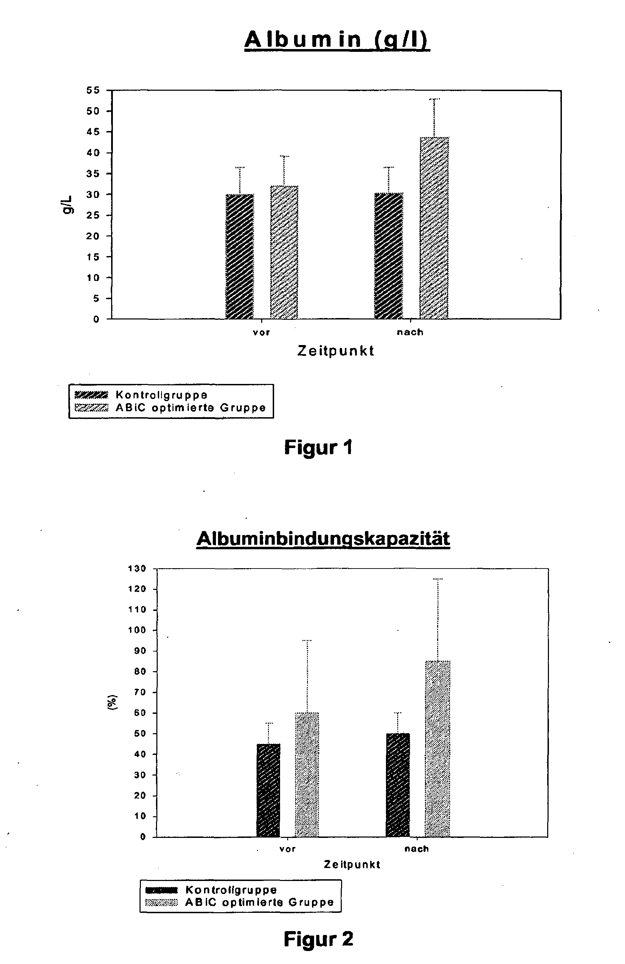

Figur 1      zeigt die Albuminkonzentrationen im Blut der beiden Versuchsgruppen vor der Therapie und nach zwei Wochen.

Figur 2      zeigt die Albuminbindungskapazitäten des Albumins im Blut der beiden Versuchsgruppen vor der Therapie und nach zwei Wochen.

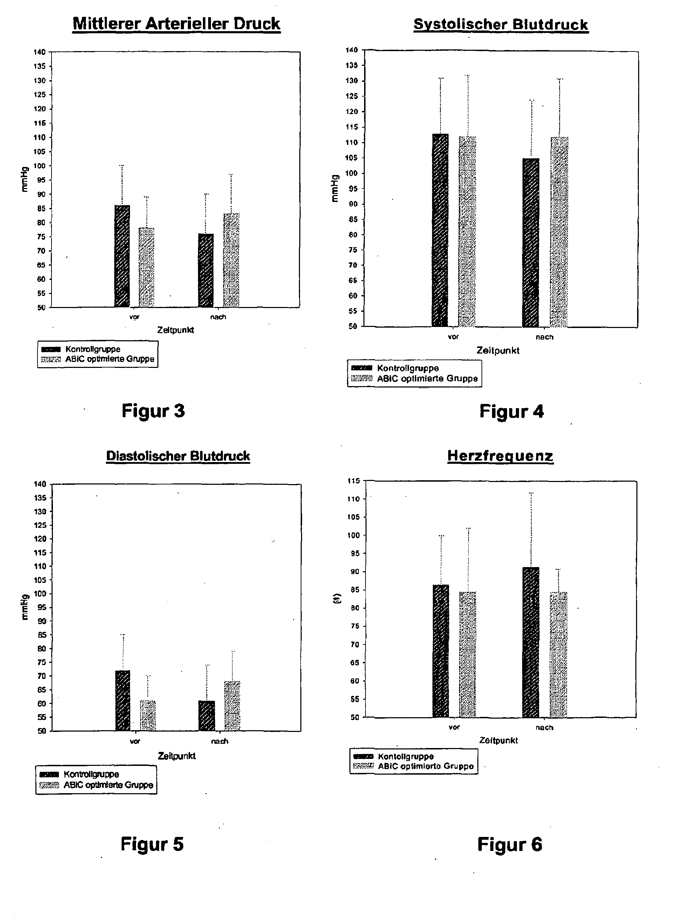

Figur 3      zeigt die Veränderung des mittleren arteriellen Füllungsdruckes der beiden Versuchsgruppen vor der Therapie und nach zwei Wochen.

Figuren 4, 5, 6      zeigen den systolischen Blutdruck, den diastolischen Blutdruck und die Herzfrequenz der beiden Versuchsgruppen vor der Therapie und nach zwei Wochen.

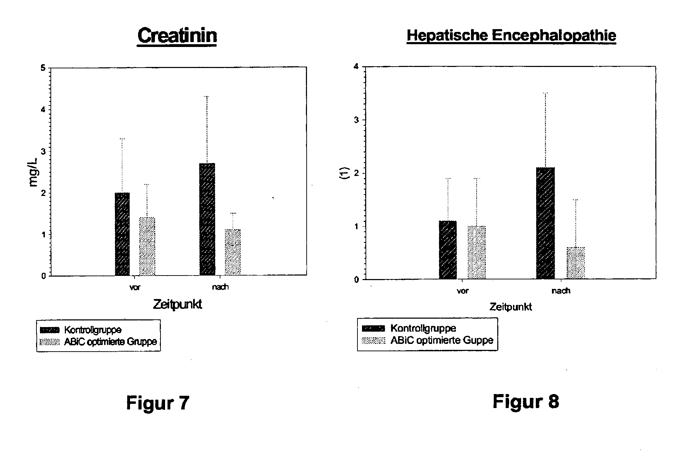

Figur 7      zeigt die Wirkung auf die Nierenfunktion, gemessen am Creatinin, der beiden Versuchsgruppen vor der Therapie und nach zwei Wochen.

Figur 8      zeigt die Wirkung auf die hepatische Enzephalopathie, die eine Folge bluthirnschrankengängiger, albuminaffiner Toxine und von Durchblutungsveränderungen ist, der beiden Versuchsgruppen vor der Therapie und nach zwei Wochen.

Die Ergebnisse des klinischen Tests zeigen, daß mit einer Verbesserung der Albuminbindungskapazität (ABiC) auch eine Verbesserung des Arteriellen Mitteldrucks einhergeht, der offenbar eher durch eine Steigerung des diastolischen Blutdruckes hervorgerufen wird als durch eine Erhöhung der Herzfrequenz. Dieses deutet medizinisch auf eine verminderte Vasodilatation hin, die bei Lebererkrankungen oft Wirkung vasodilatatorischer Substanzen mit Affinität zum Albumin ist. Die Immobilisierung derselben mit verbesserter Albuminbindung verbessert also auch die Kreislauf-, Nieren- und Hirnfunktion. Letztere kann außer aus der verbesserten Blutdrucksituation auch aus der verbesserten Bindung direkt neurotoxischer Substanzen kommen, die gleichfalls an das Albumin mit verbesserter ABiC binden.

**Patentansprüche**

1. Verfahren zur Herstellung einer wäßrigen Albuminlösung aus einer Albuminausgangslösung, welche Stabilisatormoleküle enthält, die in der Lage sind, Bindungsstellen des Albumins zu besetzen, wobei man bei dem Verfahren zur Erhöhung der Albuminbindungskapazität für andere Moleküle wenigstens einen Teil der Stabilisatormoleküle von dem Albumin der Albuminausgangslösung entfernt und von der Albuminausgangslösung abtrennt, indem man

    a) die Albuminausgangslösung mit einem festen Adsorbermaterial in Kontakt bringt, dessen Affinität für wenigstens einen Teil, vorzugsweise für alle der eingesetzten Stabilisatormoleküle höher ist als die Affinität des Albumins für die entsprechenden Stabilisatormoleküle, wobei das Adsorbermaterial ein partikuläres Aktivkohlematerial ist, welches in einer Säule oder einem Bett gepackt oder in eine Trägermatrix eingebettet ist, so dass zwischen den Partikeln des Adsorbermaterials flüssigkeitsdurchlässige Kanäle ausgebildet werden, wobei der mittlere Durchmesser der Kanäle, bezogen auf die gesamte Länge der zwischen den Partikeln des gesamten verwendeten Adsorbermaterials ausgebildeten Kanäle, mehr als 100 nm und weniger als 1000 $\mu$m beträgt und das Verfahren bei einem pH-Wert $\geq$ 5 durchgeführt wird und
    b) die Albuminlösung von dem Adsorbermaterial abtrennt.

2. Verfahren nach Anspruch 1, **dadurch gekennzeichnet, daß** das Verfahren bei einem pH-Wert im Bereich von 5 bis 9, vorzugsweise im Bereich von 6 bis 8, besonders bevorzugt im Bereich von 6,9 bis 7,5 durchgeführt wird.

3. Verfahren nach einem der vorangegangenen Ansprüche, **dadurch gekennzeichnet, daß** das Albumin humanes Serumalbumin (HSA) ist.

4. Verfahren nach einem der vorangegangenen Ansprüche, **dadurch gekennzeichnet, daß** die zu entfernenden Stabilisatormoleküle N-Acetyltryptophan und/oder Octansäure oder deren Anionen umfassen.

5. Verfahren nach einem der vorangegangenen Ansprüche, **dadurch gekennzeichnet, daß** das Inkontaktbringen der Albuminausgangslösung und des Adsorbermaterials in Stufe a) durch Hindurchleiten der Albuminausgangslösung durch eine das Adsorber material enthaltende Säule (Chromatographiesäule) oder durch ein von dem Adsobermaterial gebildetes Bett erfolgt.

6. Verfahren nach einem der vorangegangenen Ansprüche, **dadurch gekennzeichnet, daß** das Abtrennen der Albuminlösung von dem Adsorbermaterial in Stufe b) durch Filtration der Albuminlösung durch einen Partikelfilter erfolgt, wobei der Partikelfilter so ausgewählt ist, daß er Albuminmoleküle hindurchtreten läßt und das feste Adsorbermaterial zurückhält.

7. Verfahren nach einem der vorangegangenen Ansprüche, **dadurch gekennzeichnet, daß** die Stufen a) und b) mehrmals, vorzugsweise 2 bis 6 mal wiederholt werden, wobei jeweils die in Stufe b) gewonnene behandelte Albuminlösung in die Stufe a) zurückgeführt wird und in der Stufe a) jeweils durch Entfernung von Stabilisatormolekülen regeneriertes und/oder frisches Adsorbermaterial eingesetzt wird.

8. Verfahren nach einem der vorangegangenen Ansprüche, **dadurch gekennzeichnet, daß** die Menge an eingesetztem Adsorbermaterial im Verhältnis zur Albuminkonzentration in der Albuminausgangslösung und/oder die Kontaktzeit zwischen Albuminausgangslösung und Adsorbermaterial in Stufe a) so ausgewählt sind, daß die Albuminbindungskapazität (ABiC) der hergestellten Albuminlösung, gemessen nach Klammt et al., wenigstens 60 %, vorzugsweise wenigstens 70 %, besonders bevorzugt wenigstens 80 %, ganz besonders bevorzugt mindestens 90 % beträgt.

9. Verfahren nach einem der vorangegangenen Ansprüche, **dadurch gekennzeichnet, daß** die Menge an eingesetztem Adsorbermaterial im Verhältnis zur Albuminkonzentration in der Albuminausgangslösung und/oder die Kontaktzeit zwischen Albuminausgangslösung und Adsorbermaterial in Stufe a) so ausgewählt sind, daß die Konzentrationen an gebundenen und ungebundenen Stabilisatormolekülen, insbesondere an N-Acetyltryptophan und/oder Octansäure oder deren Anionen in der Albuminausgangslösung auf weniger als 70 %, vorzugsweise weniger als 50 %, besonders bevorzugt auf weniger als 30 %, ganz besonders bevorzugt auf weniger als 10 % ihrer Ausgangskonzentrationen gesenkt werden.

10. Verfahren nach einem der vorangegangenen Ansprüche, **dadurch gekennzeichnet, daß** das Adsorbermaterial ein partikuläres Material ist, welches in einer Säule oder einem Bett gepackt oder in eine Trägermatrix eingebettet ist, so daß zwischen den Partikeln des Adsorbermaterials flüssigkeitsdurchlässige Kanäle ausgebildet werden, wobei der mittlere Durchmesser der Kanäle, bezogen auf die gesamte Länge der zwischen den Partikeln des gesamten verwendeten Adsorbermaterials ausgebildeten Kanäle weniger als 500 $\mu$m, bevorzugt weniger als 300 $\mu$m, besonders bevorzugt weniger als 200 $\mu$m, noch bevorzugter weniger als 100 $\mu$m beträgt.

11. Verfahren nach Anspruch 10, **dadurch gekennzeichnet, daß** der mittlere Durchmesser der Kanäle, bezogen auf die gesamte Länge der zwischen den Partikeln des gesamten verwendeten Adsorbermaterials ausgebildeten Kanäle, mehr als 100 nm und weniger als 60 $\mu$m und ganz besonders bevorzugt weniger als 10 $\mu$m beträgt.

12. Verfahren nach einem der vorangegangenen Ansprüche, **dadurch gekennzeichnet, daß** die minimale Kontaktzeit zwischen Albuminausgangslösung und Adsorbermaterial in Stufe a) so ausgewählt ist, daß

$$\text{dm [µm] / 10 [µm/min]} \leq \text{Kontaktzeit [min]} \leq \text{dm [µm] / 0,1 [µm/min]},$$

wobei dm der mittlere Durchmesser der Kanäle, bezogen auf die gesamte Länge der zwischen den Partikeln des gesamten verwendeten Adsorbermaterials ausgebildeten Kanäle, ist.

13. Verfahren nach einem der vorangegangenen Ansprüche, **dadurch gekennzeichnet, daß** die minimale Kontaktzeit zwischen Albuminausgangslösung und Adsorbermaterial in Stufe a) so ausgewählt ist, daß

$$\text{dm [µm] / 4 [µm/min]} \leq \text{Kontaktzeit [min]} \leq \text{dm [µm] / 0,3 [µm/min]},$$

wobei dm der mittlere Durchmesser der Kanäle, bezogen auf die gesamte Länge der zwischen den Partikeln des gesamten verwendeten Adsorbermaterials ausgebildeten Kanäle, ist.

14. Verfahren nach einem der vorangegangenen Ansprüche, **dadurch gekennzeichnet, daß** die Aktivkohle eine Melassezahl (nach IUPAC) von 100 bis 400, vorzugsweise von 200 bis 300 aufweist.

15. Verfahren nach einem der vorangegangenen Ansprüche, **dadurch gekennzeichnet, daß** die Aktivkohle eine Methylenblau-Adsorption (nach IUPAC) von 1 bis 100 g/100 g Aktivkohle, vorzugsweise von 10 bis 30 g/100 g Aktivkohle aufweist.

16. Verfahren nach einem der vorangegangenen Ansprüche, **dadurch gekennzeichnet, daß** die Aktivkohle eine Iodzahl (nach IUPAC) von 500 bis 3000, vorzugsweise von 800 bis 1500 aufweist.

17. Verfahren nach einem der vorangegangenen Ansprüche, **dadurch gekennzeichnet, daß** die Aktivkohle eine totale innere Oberfläche (BET) (nach IUPAC) von 100 bis 5000 m$^2$/g Aktivkohle, vorzugsweise von 800 bis 1400 m$^2$/g Aktivkohle aufweist.

18. Adsorbermaterial mit den in einem der vorangegangenen Ansprüche beschriebenen Merkmalen für die Durchführung des Verfahrens nach einem der vorangegangenen Ansprüche.

**19.** Verwendung eines Adsorbermaterials nach Anspruch 18 zur Durchführung eines Verfahrens nach einem der Ansprüche 1 bis 17.

**20.** Verwendung einer wäßrigen Albuminlösung, hergestellt nach einem der Ansprüche 1 bis 17, zur Herstellung eines Mittels für die Behandlung von Hypoalbuminaemie.

**21.** Verwendung einer wäßrigen Albuminlösung, hergestellt nach einem der Ansprüche 1 bis 17, zur Herstellung eines Volumenersatzmittels bzw. Plasmaexpanders oder einer Lösung für die extrakorporale Blutreinigung mittels Apherese oder Albumindialyse.

**22.** Verwendung einer wäßrigen Albuminlösung, hergestellt nach einem der Ansprüche 1 bis 17, zur Herstellung eines Mittels für die Verbesserung der Kreislauf-, Nieren-und/oder Hirnfunktion eines Patienten.

**23.** Albuminlösung, hergestellt nach einem Verfahren gemäß einem der Ansprüche 1 bis 17.

**Claims**

**1.** A method for producing an aqueous albumin solution from a starting albumin solution which contains stabilizer molecules which are capable of occupying binding sites of the albumin, wherein in a method for increasing the albumin binding capacity for other molecules, at least a portion of the stabilizer molecules is removed from the albumin of the starting albumin solution and separated from the starting albumin solution, wherein:

a) the starting albumin solution is brought into contact with a solid adsorption material the affinity of which for at least a portion, preferably all of the stabilizer molecules used is higher than the affinity of the albumin for the corresponding stabilizer molecules;
wherein the adsorption material is a particulate activated charcoal material which is packed in a column or a bed or a support matrix so that fluid-carrying channels are formed between the particles of adsorption material, wherein the mean diameter of the channels, relative to the total length of the channels formed between the particles of all of the adsorption material employed, is more than 100 nm and less than 1000 $\mu$m; and
the method is carried out at a pH of $\geq$ 5; and
b) the albumin is separated from the adsorption material.

**2.** A method according to claim 1, **characterized in that** the method is carried out at a pH in the range 5 to 9, preferably in the range 6 to 8, particularly preferably in the range 6.9 to 7.5.

**3.** A method according to one of the preceding claims, **characterized in that** the albumin is human serum albumin (HSA).

**4.** A method according to one of the preceding claims, **characterized in that** the stabilizer molecules to be removed include N-acetyl tryptophan and/or octanoic acid or anions thereof.

**5.** A method according to one of the preceding claims, **characterized in that** the starting albumin solution and the adsorption material are brought into contact in step a) by feeding the starting albumin solution through a column containing the adsorption material (chromatographic column) or through a bed formed by the adsorption material.

**6.** A method according to one of the preceding claims, **characterized in that** separation of the albumin solution from the adsorption material in step b) is carried out by filtering the albumin solution through a particle filter, wherein the particle filter is selected so that the albumin molecules can pass through and the solid adsorption material is retained.

**7.** A method according to one of the preceding claims, **characterized in that** steps a) and b) are repeated several times, preferably 2 to 6 times, wherein in each case the treated albumin solution obtained in step b) is fed back to step a), and in step a) regenerated and/or fresh adsorption material is used for the removal of stabilizer molecules.

**8.** A method according to one of the preceding claims, **characterized in that** the amount of adsorption material used compared with the albumin concentration in the starting albumin solution and/or the contact time between the starting albumin solution and the adsorption material in step a) is selected so that the albumin binding capacity (ABiC) of the albumin solution produced, measured in accordance with Klammt et al, is at least 60%, preferably at least 70%, particularly preferably at least 80% and more particularly preferably at least 90%.

9.  A method according to one of the preceding claims, **characterized in that** the amount of adsorption material used compared with the albumin concentration in the starting albumin solution and/or the contact time between the starting albumin solution and the adsorption material in step a) is selected so that the concentration of bound and unbound stabilizer molecules, in particular N-acetyl tryptophan and/or octanoic acid or their anions, in the starting albumin solution is reduced to less than 70%, preferably less than 50%, more preferably less than 30% and particularly preferably less than 10% of its starting concentration.

10. A method according to one of the preceding claims, **characterized in that** the adsorption material is a particulate material which is packed in a column or a bed or a support matrix so that fluid-carrying channels are formed between the particles of adsorption material, wherein the mean diameter of the channels, relative to the total length of the channels formed between the particles of all of the adsorption material employed, is less than 500 μm, preferably less than 300 μm and preferably less than 200 μm, still more preferably less than 100 μm.

11. A method according to claim 10, **characterized in that** the mean diameter of the channels, taken over the total length of the channels formed between the particles of all of the adsorption material, is more than 100 nm and less than 60 μm, particularly preferably less than 10 μm.

12. A method according to one of the preceding claims, **characterized in that** the minimum contact time between the starting albumin solution and the adsorption material in step a) is selected so that:

$$\text{dm [μm] / 10 [μm/min]} \leq \text{contact time [min]} \leq \text{dm [μm] / 0.1 [μm/min]},$$

wherein "dm" means the mean channel diameter, taken over the total length of the channels formed between the particles of all of the adsorption material.

13. A method according to one of the preceding claims, **characterized in that** the minimum contact time between the starting albumin solution and the adsorption material in step a) is selected so that:

$$\text{dm [μm] / 4 [μm/min]} \leq \text{contact time [min]} \leq \text{dm [μm] / 0.3 [μm/min]},$$

wherein "dm" means the mean channel diameter, taken over the total length of the channels formed between the particles of all of the adsorption material.

14. A method according to one of the preceding claims, **characterized in that** the activated charcoal has a molasses number (IUPAC) of 100 to 400, preferably 200 to 300.

15. A method according to one of the preceding claims, **characterized in that** the activated charcoal has a methylene blue adsorption (IUPAC) of 1 to 100 g/100 g of activated charcoal, preferably 10 to 30 g/100 g of activated charcoal.

16. A method according to one of the preceding claims, **characterized in that** the activated charcoal has an iodine number (IUPAC) of 500 to 3000, preferably 800 to 1500.

17. A method according to one of the preceding claims, **characterized in that** the activated charcoal has a total internal surface area (BET) (IUPAC) of 100 to 5000 $m^2$/g of activated charcoal, preferably 800 to 1400 $m^2$/g of activated charcoal.

18. An adsorption material with the features defined in one of the preceding claims, to carry out the method according to one of the preceding claims.

19. Use of an adsorption material according to claim 18, to carry out a method according to one of claims 1 to 17.

20. Use of an aqueous albumin solution produced in accordance with one of claims 1 to 17, for the production of a means for treatment of hypoalbuminaemia.

**21.** Use of an aqueous albumin solution produced in accordance with one of claims 1 to 18, for the production of a volume replacement means or plasma expander or a solution for extracorporal blood purification using apheresis or albumin dialysis.

**22.** Use of an aqueous albumin solution produced in accordance with one of claims 1 to 17, for the production of a means for improving the circulation, kidney and/or brain function of a patient.

**23.** An albumin solution, produced in accordance with a method according to one of claims 1 to 17.

**Revendications**

**1.** Procédé de préparation d'une solution aqueuse d'albumine à partir d'une solution d'albumine de départ, qui contient des molécules stabilisantes, qui sont à même d'occuper des sites de liaison de l'albumine, on élimine de l'albumine de la solution d'albumine de départ, dans le procédé destiné à augmenter la capacité de liaison de l'albumine à d'autres molécules, au moins une partie des molécules stabilisantes, et on la sépare de la solution d'albumine de départ, dans lequel

a) on met la solution d'albumine de départ en contact avec un matériau adsorbant solide, dont l'affinité pour au moins une partie, de préférence pour la totalité des molécules stabilisantes utilisées, est supérieure à l'affinité de l'albumine pour les molécules stabilisantes correspondantes,
le matériau adsorbant étant un matériau à base de charbon actif particulaire, dont est garni une colonne ou un lit, ou est incorporé dans une matrice support de telle sorte que des canaux perméables aux liquides se forment entre les particules du matériau adsorbant, le diamètre moyen des canaux, rapporté à la longueur totale des canaux formés entre les particules de la totalité du matériau adsorbant utilisé, étant supérieur à 100 nm et inférieur à 1000 $\mu$m, et
le procédé étant mis en oeuvre à un pH $\geq$ 5, et
b) on sépare la solution d'albumine du matériau adsorbant.

**2.** Procédé selon la revendication 1, **caractérisé en ce que** le procédé est mis en oeuvre à un pH compris dans la plage de 5 à 9, de préférence dans la plage de 6 à 8, d'une manière particulièrement préférée dans la plage de 6,9 à 7,5.

**3.** Procédé selon l'une des revendications précédentes, **caractérisé en ce que** l'albumine est la sérumalbumine humaine (HSA).

**4.** Procédé selon l'une des revendications précédentes, **caractérisé en ce que** les molécules stabilisantes à éliminer comprennent le N-acétyltryptophane et/ou l'acide octanoïque ou ses anions.

**5.** Procédé selon l'une des revendications précédentes, **caractérisé en ce que** la mise en contact de la solution d'albumine de départ et du matériau adsorbant dans l'étape a) est réalisé par passage de la solution d'albumine de départ à travers une colonne contenant le matériau adsorbant (colonne chromatographique) ou à travers un lit formé du matériau adsorbant.

**6.** Procédé selon l'une des revendications précédentes, **caractérisé en ce que** la séparation de la solution d'albumine du matériau adsorbant de l'étape b) s'effectue par filtration de la solution d'albumine à travers un filtre particulaire, le filtre particulaire étant choisi de façon à laisser passer les molécules d'albumine et à retenir le matériau adsorbant solide.

**7.** Procédé selon l'une des revendications précédentes, **caractérisé en ce que** les étapes a) et b) sont répétées plusieurs fois, de préférence 2 à 6 fois, la solution d'albumine traitée, obtenue dans l'étape b) étant chaque fois renvoyée dans l'étape a), et, dans l'étape a), on utilise dans chaque cas le matériau adsorbant régénéré par élimination des molécules stabilisantes, et/ou frais.

**8.** Procédé selon l'une des revendications précédentes, **caractérisé en ce que** la quantité du matériau adsorbant utilisé, rapportée à la concentration de l'albumine dans la solution d'albumine de départ, et/ou le temps de contact entre la solution d'albumine de départ et le matériau adsorbant de l'étape a), sont choisis de façon que la capacité de liaison de l'albumine (ABiC) de la solution d'albumine préparée, mesurée d'après Klammt et al., soit d'au moins 60 %, de préférence d'au moins 70 %, d'une manière particulièrement préférée d'au moins 80 % et d'une manière tout particulièrement préférée d'au moins 90 %.

9. Procédé selon l'une des revendications précédentes, **caractérisé en ce que** la quantité du matériau adsorbant utilisé, rapportée à la concentration de l'albumine dans la solution d'albumine de départ, et/ou le temps de contact entre la solution d'albumine de départ et le matériau adsorbant de l'étape a), sont choisis de telle sorte que les concentrations des molécules stabilisantes liées et non liées, en particulier du N-acétyltryptophane et/ou de l'acide octanoïque ou de ses anions, dans la solution d'albumine de départ, soit abaissée à une valeur inférieure à 70 %, de préférence inférieure à 50 %, d'une manière particulièrement préférée inférieure à 30 %, d'une manière tout particulièrement préférée inférieure à 10 % de leur concentration de départ.

10. Procédé selon l'une des revendications précédentes, **caractérisé en ce que** le matériau adsorbant est un matériau particulaire dont est garni une colonne ou un lit ou est incorporé dans une matrice support, de telle sorte qu'il se forme des canaux perméables aux liquides entre les particules du matériau adsorbant, le diamètre moyen des canaux, rapporté à la longueur totale des canaux formés entre les particules de la totalité du matériau adsorbant utilisé, étant inférieur à 500 $\mu$m, de préférence inférieur à 300 $\mu$m, d'une manière particulièrement préférée inférieur à 200 $\mu$m, d'une manière encore plus préférée inférieur à 100 $\mu$m.

11. Procédé selon la revendication 10, **caractérisé en ce que** le diamètre moyen des canaux, rapporté à la longueur totale des canaux formés entre les particules de la totalité du matériau adsorbant utilisé, est supérieur à 100 nm et inférieur à 60 $\mu$m, et d'une manière tout particulièrement préférée inférieur à 10 $\mu$m.

12. Procédé selon l'une des revendications précédentes, **caractérisé en ce que** le temps de contact minimum entre la solution d'albumine de départ et le matériau adsorbant de l'étape a) est choisi de telle sorte que

$$\mathrm{dm}[\mu m]/10[\mu m/min] \leq \text{temps de contact}[min] \leq \mathrm{dm}[\mu m]/0{,}1[\mu m/min]$$

dm étant le diamètre moyen des canaux, rapporté à la longueur totale des canaux formés entre les particules de la totalité du matériau adsorbant utilisé.

13. Procédé selon l'une des revendications précédentes, **caractérisé en ce que** le temps de contact minimum entre la solution d'albumine de départ et le matériau adsorbant de l'étape a) est choisi de telle sorte que

$$\mathrm{dm}[\mu m]/4[\mu m/min] \leq \text{temps de contact}[min] \leq \mathrm{dm}[\mu m]/0{,}3[\mu m/min]$$

dm étant le diamètre moyen des canaux, rapporté à la longueur totale des canaux formés entre les particules de la totalité du matériau adsorbant utilisé.

14. Procédé selon l'une des revendications précédentes, **caractérisé en ce que** le charbon actif présente un indice de mélasse (d'après l'IUPAC) de 100 à 400, de préférence de 200 à 300.

15. Procédé selon l'une des revendications précédentes, **caractérisé en ce que** le charbon actif présente une adsorption du bleu de méthylène (d'après l'IUPAC) de 1 à 100 g/100 g de charbon actif, de préférence de 10 à 30 g/100 g de charbon actif.

16. Procédé selon l'une des revendications précédentes, **caractérisé en ce que** le charbon actif présente un indice d'iode (d'après l'IUPAC) de 500 à 3000, de préférence de 800 à 1500.

17. Procédé selon l'une des revendications précédentes, **caractérisé en ce que** le charbon actif présente une aire interne totale (BET) (d'après l'IUPAC) de 100 à 5000 m$^2$/g de charbon actif, de préférence de 800 à 1400 m$^2$/g de charbon actif.

18. Matériau adsorbant présentant les caractéristiques décrites dans l'une des revendications précédentes pour la mise en oeuvre du procédé selon l'une des revendications précédentes.

**19.** Utilisation d'un matériau adsorbant selon la revendication 18 pour la mise en oeuvre d'un procédé selon l'une des revendications 1 à 17.

**20.** Utilisation d'une solution aqueuse d'albumine préparée selon l'une des revendications 1 à 17 pour la fabrication d'un moyen destiné au traitement d'une hypoalbuminémie.

**21.** Utilisation d'une solution aqueuse d'albumine préparée selon l'une des revendications 1 à 17 pour fabriquer un succédané volumique ou un expanseur plasmatique, ou une solution pour la purification extracorporelle du sang par aphérèse ou dialyse à l'albumine.

**22.** Utilisation d'une solution aqueuse d'albumine, préparée selon l'une des revendications 1 à 17, pour fabriquer un produit destiné à améliorer la fonction circulatoire, rénale et/ou cérébrale d'un patient.

**23.** Solution d'albumine, préparée par un procédé selon l'une des revendications 1 à 17.

EP 1 879 909 B1

# A lb u m in (g /l)

**Figur 1**

# Albuminbindungskapazität

**Figur 2**

## Mittlerer Arterieller Druck

**Figur 3**

## Systolischer Blutdruck

**Figur 4**

## Diastolischer Blutdruck

**Figur 5**

## Herzfrequenz

**Figur 6**

## Creatinin

**Figur 7**

## Hepatische Encephalopathie

**Figur 8**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040217055 A

**In der Beschreibung aufgeführte Nicht-Patentliteratur**

- *Cochrane Metaanalyse im BMJ,* 1998, vol. 317, 235-240
- **J. PETERS JR.** All about Albumin. Academic Press, 1996
- **PACIFICI GM ; VIANI A.** *Methods of Determining Plasma and Tissue Binding of Drugs, Clin Pharmacokinet,* 1992, vol. 23 (6), 449-468
- **KLAMMT S ; BRINKMANN B ; MITZNER S ; MUNZERT E ; LOOCK J ; STANGE J ; EMMRICH J ; LIEBE S.** Albumin Binding Capacity (ABiC) is reduced in commercially available Human Serum Albumin preparations with stabilizers. *Zeitschrift für Gastroenterologie,* 2001, vol. 39, 24-27
- **GOODMAN DS.** *Science,* 1996, vol. 125, 1957
- **CHEN et al.** *Journal of Biological Chemistry,* 25. Januar 1967, vol. 212 (2), 173-181